# EUROPÄISCHE PATENTSCHRIFT

(11) **EP 0 867 432 B1**
(45) Veröffentlichungstag und Bekanntmachung des Hinweises auf die Patenterteilung: **09.06.2004**
(21) Anmeldenummer: 98105200.4
(22) Anmeldetag: 23.03.1998
(51) Int. Cl.: C07D 215/26, A61K 31/47, C07D 405/12, C07D 401/12

(54) **Benzyloxy-substituierte, anellierte N-Heterocyclen, Verfahren zu ihrer Herstellung und ihre Verwendung als Bradykininrezeptorantagonisten**
Fused N-heterocyclic compounds substituted with benzyloxy, process for their preparation and their use as bradykinin receptor antagonists
Composés N-hétérocycliques condensés substitués avec benzyloxy, procédé de préparation et leur utilisation comme antagonistes des récepteurs de la bradykinine

(30) Priorität: 27.03.1997 DE 19712960
(43) Veröffentlichungstag der Anmeldung: 30.09.1998
(73) Patentinhaber: Aventis Pharma Deutschland GmbH, 65929 Frankfurt am Main (DE)
(72) Erfinder: Heitsch, Holger, Dr., 55252 Mainz-Kastel (DE); Wagner, Adalbert, Dr., 86368 Gersthofen (DE); Wirth, Klaus, Dr., 65830 Kriftel (DE); Schölkens, Bernward, Prof. Dr., 65779 Kelkheim (DE)

(56) Entgegenhaltungen:
- EP-A- 0 622 361
- EP-A- 0 796 848
- EP-A- 0 808 628
- WO-A-96/13485
- WO-A-96/40639

## Beschreibung

Aus EP-A-6 22 361, WO 96-13485 und WO 96-40639 sowie den prioritätsälteren, noch nicht offengelegten Patentanmeldungen P 19610784.9 und P 19609827.0 sind Benzyloxy-substituierte, anellierte N-Heterozyklen und ihre Verwendung als Bradykinin-Antagonisten bekannt.

Die vorliegende Erfindung betrifft neue Benzyloxy-substituierte, anellierte N-Heterozyklen mit hoher Affinität zum Bradykinin-B₂-Rezeptor and verbesserter Pharmakokinetik.

Die Verbindungen werden durch die Formel (I) beschrieben, 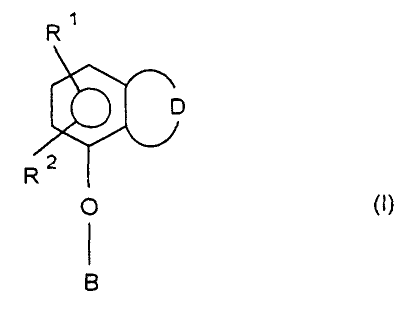 in welcher die Symbole die folgende Bedeutung haben:
- D: steht für einen Rest der Formel (II) oder (III): 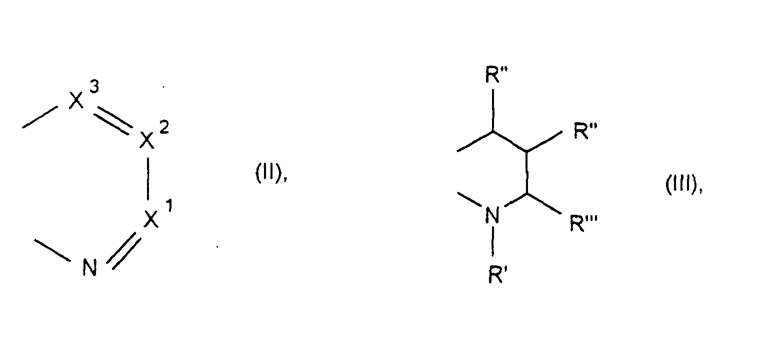
- B: ein Rest der Formel (IX), 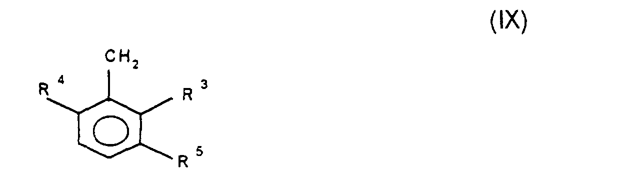
- X¹: C-CH₃,
- X²: C-H,
- X³: C-H,
- R': Methyl,
- R": Wasserstoff,
- R''': Methyl,
- R¹, R²: Wasserstoff,
- R³, R⁴: gleich oder verschieden
1. Chlor,
2. Cyano,
3. Methyl,
4. O-Methyl,
5. S-Methyl,
6. OH,
7. Tetrazolyl,
8. CONH₂,
- R⁵: 1. Nitro,
2. Amino,
3. ein Rest der Formel (X) 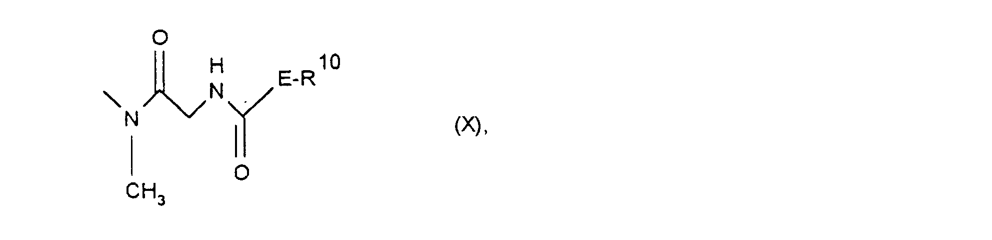
4. ein Rest der Formel (V) 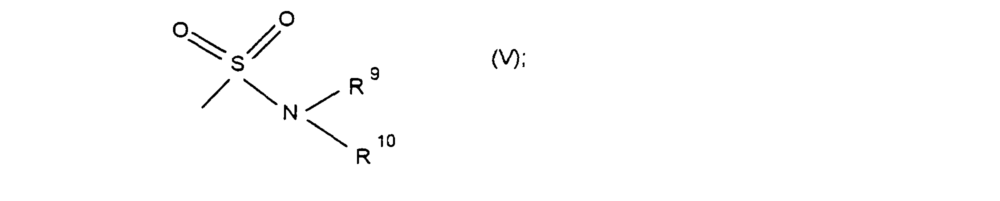
- R⁹: 1. Wasserstoff,
2. Methyl, Ethyl, n- und i-Propyl und -Butyl,
3. Benzyl
- E: 1. (C₂-C₅)-Alkandiyl,
2. (C₁-C₇)-Alkandiyl,
3. -(CH₂)ₘ-Tₒ-(CH₂)ₙ-,
wobei die unter 1.-3. aufgeführten Reste gegebenenfalls substituiert sind mit einer Gruppe aus der Reihe OR¹², CO₂R⁹, NR¹³R¹⁴ oder CONR¹³R¹⁴:
- T: 1.O,
2. NH;
- m,n: gleich oder verschieden eine Zahl von 0 bis 3;
- o: eine Zahl von 0 oder 1;
- R¹⁰: 1. Wasserstoff,
2. (C₁-C₅)-Alkyl,
3. Phenyl,
4. Benzyl,
5. (C₅-C₇)-Heteroaryl, bevorzugt Furyl und Pyridyl,
wobei 3., 4. und 5. gegebenenfalls mit einer oder 2 Gruppen aus der Reihe (C₁-C₅)-Alkyl, (C₁-C₅)-Alkoxy, CF₃, OCF₃, NR¹³R¹⁴, NR¹³CO-R¹⁶, CO₂R⁹ substituiert sein können;
- R¹¹: 1. CF₃,
2. OCF₃;
- R¹², R¹³: gleich oder verschieden
1. Wasserstoff,
2. Methyl, Ethyl,
3. Phenyl,
4. Benzyl;
- R¹⁴: 1. Wasserstoff,
2. C(O)-O-C(CH₃)₃
3. C(O)-O-CH₂-Phenyl
- R¹⁶: 1. Methyl, Ethyl,
2. Phenyl,
3. (C₅-C₇)-Heteroaryl
wobei diese Reste gegebenenfalls mit einer oder zwei Gruppen aus der Reihe NR¹³R¹⁴ oder CO₂R⁹ substituiert sein können;
sowie deren physiologisch verträgliche Salze;

Ganz besonders bevorzugt sind Verbindungen der Formel (I), in denen D für die Formel (II) steht und die übrigen Reste, Variablen wie oben definiert sind.

Die Erfindung betrifft weiterhin ein Verfahren zur Herstellung von Verbindungen der Formel (I), das dadurch gekennzeichnet ist, daß man
α.
   a) eine Verbindung der Formel (XI) 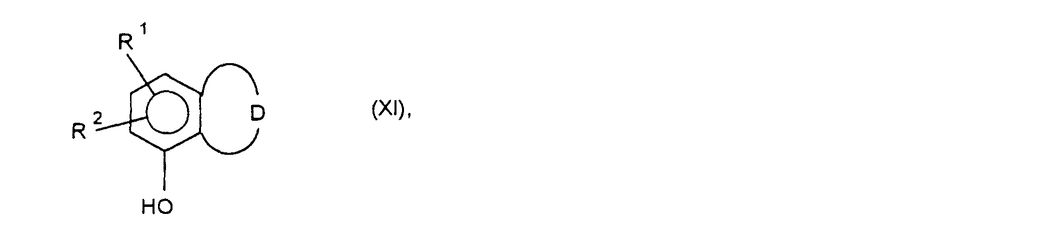 worin R¹, R² und D wie oben definiert sind, in Gegenwart von Metallhydriden, wie Lithium-, Kalium- oder Natriumhydrid, oder Alkalimetallcarbonaten, wie Na₂CO₃, K₂CO₃ oder Cs₂CO₃ in einem inerten Lösungsmittel, wie DMF und DMSO, bei Temperaturen von 0°C bis 60°C, bevorzugt bei Raumtemperatur, mit einer Verbindung der Formel (XII) 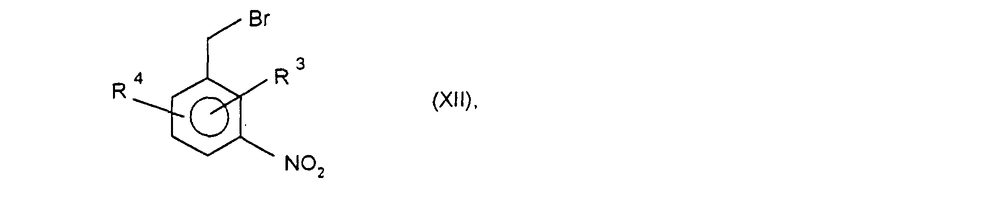 worin R³ und R⁴ wie oben in Formel (I) definiert sind, zu einer Verbindung der Formel (XIII) 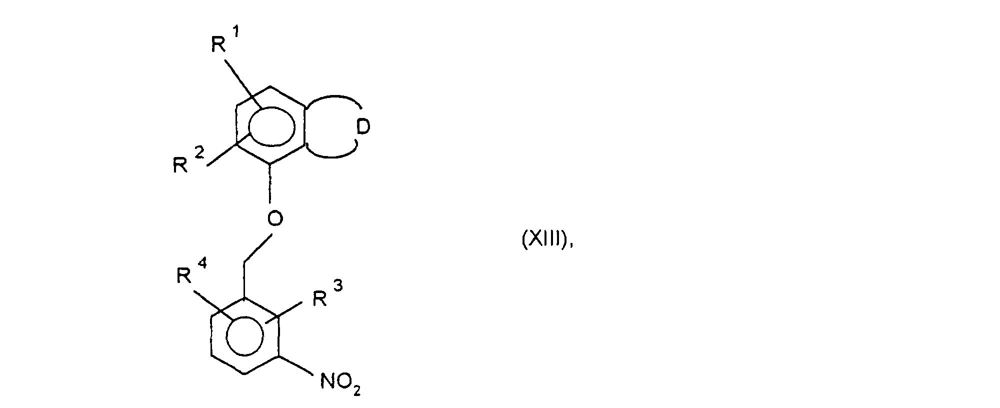 worin R¹, R², R³, R⁴ und D wie oben definiert sind, umsetzt;
   b) die Verbindung der Formel (XIII) mit Hilfe von Übergangsmetallhalogeniden, bevorzugt SnCl₂ und FeCl_{3,} zu einer Verbindung der Formel (XIV) reduziert 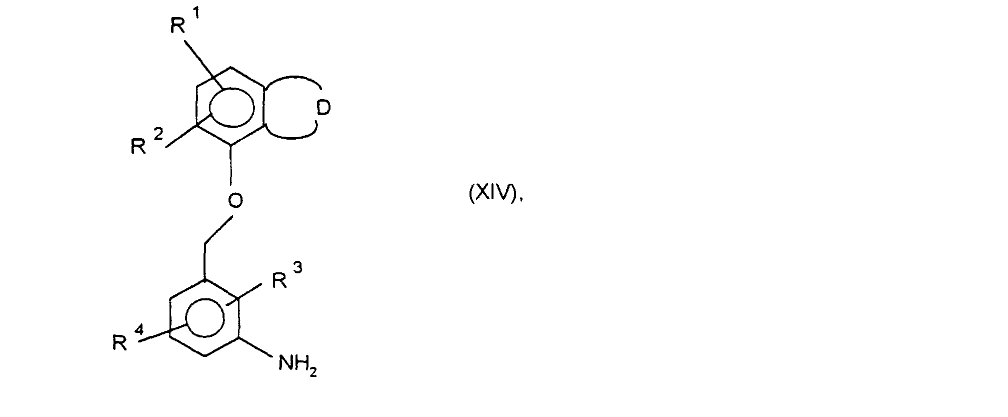 worin R¹, R², R³, R⁴ und D wie oben definiert sind;
   c) eine Verbindung der Formel (XIV) mit aktivierten, geeignet geschützten Aminocarbonsäurederivaten von A (= A-Prot), bevorzugt den Säurechloriden der Phthaloyl-geschützten Aminocarbonsäurederivate von A, in inerten Lösungsmitteln wie CH₂Cl₂ oder N-Methylpyrrolidon, gegebenenfalls durch Zugabe von DMAP, in Gegenwart einer Base wie z. B. Pyridin umsetzt und so eine Verbindung der Formel (XV) erhält 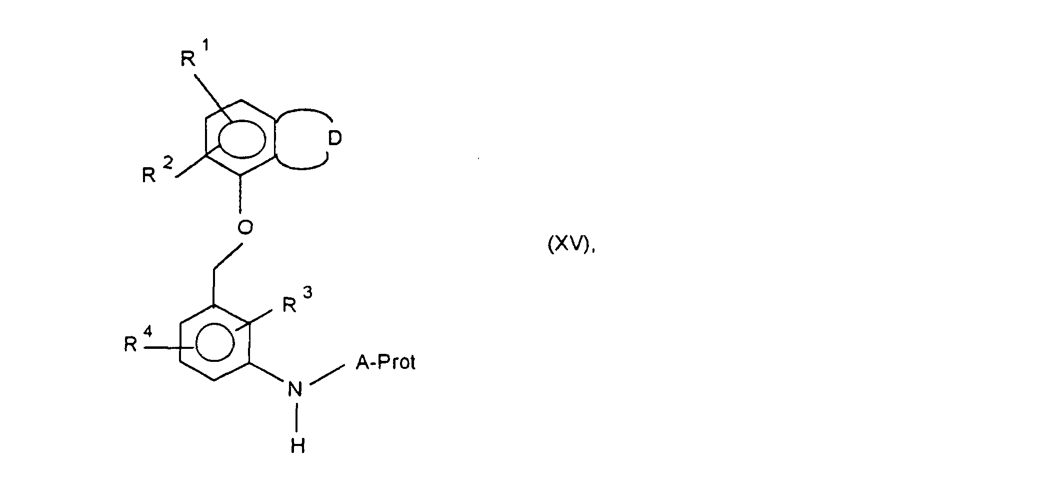 worin A, R¹, R², R³, R⁴ und D wie oben definiert sind, und Prot für eine Aminoschutzgruppe steht, wie in T.W. Greene "Protective Groups in Organic Synthesis", Verlag John Wiley, 2. Auflage 1991, beschrieben (z.B. Phthaloyl, Benzyl oder Paramethoxybenzyl);
   d) eine Verbindung der Formel (XV) nach erfolgter Einwirkung von Alkalihydriden, Alkalicarbonaten oder Alkoholaten in inerten Lösungsmitteln, bevorzugt DMF oder N-Methylpyrrolidon, gefolgt von einer Behandlung mit R⁹X, wobei R⁹ wie oben definiert ist und X eine Abgangsgruppe, z.B. Halogen, Mesylat oder Tosylat, bedeutet, umsetzt, wobei man eine Verbindung der Formel (XVI) erhält 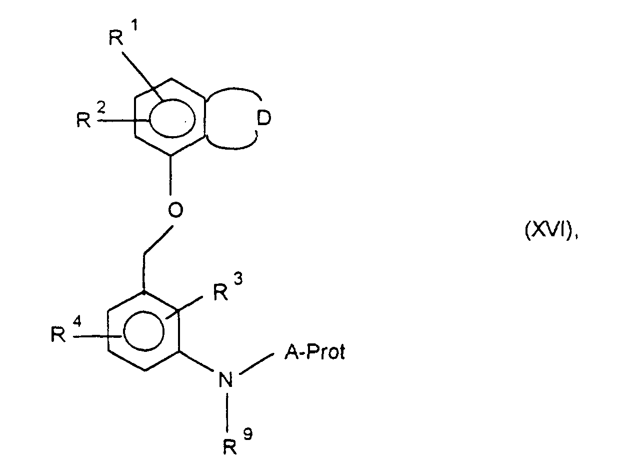 worin R¹, R², R³, R⁴, R⁹, D und A wie oben und Prot wie in Formel (XV) definiert sind;
   e) eine Verbindung der Formel (XVI) durch Hydrazinolyse in Ethanol, im Falle der Phthaloylgruppe als Schutzgruppe Prot, bei einer Temperatur von 20C bis zum Siedepunkt in eine Verbindung der Formel (XVII) 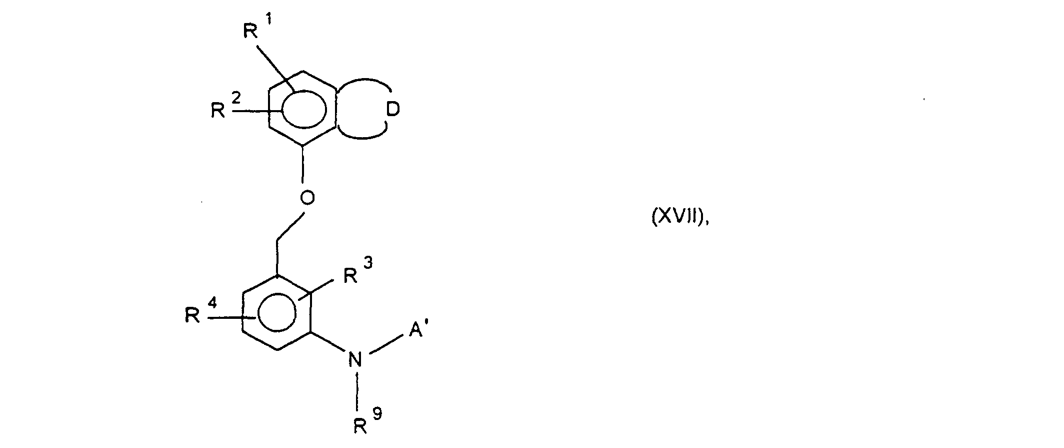 worin R¹, R², R³, R⁴, R⁹, und D wie oben definiert sind und A' für einen Rest einer Aminocarbonsäure, steht überführt;
   f1) eine Verbindung der Formel (XVII) mit aktivierten Carbonsäure- und Sulfonsäure-Derivaten R¹⁰-E-Y-OH, worin R¹⁰, E und Y wie oben definiert sind, in konventionellen organischen Lösungsmitteln wie CH₂Cl₂, Dioxan, THF oder DMF in Gegenwart einer anorganischen oder organischen Base bei einer Temperatur von 0°C bis Rückfluß zu einer Verbindung der Formel (I), worin R¹, R², R³, R⁴, R⁹, R¹⁰, A, D, E die obengenannte Bedeutung haben, B ein Rest der Formel (VIII) und R⁵ ein Rest der Formel IV ist, umsetzt,
      oder
   f2) eine Verbindung der Formel (XVII) mit einem Amin oder einem Alkohol R¹⁰-E-NH₂ bzw. R¹⁰-E-OH, bevorzugt bei einer Temperatur von 0°C bis Raumtemperatur in inerten Lösungsmitteln wie Dichlormethan oder Dimethoxyethan zu einer Verbindung der Formel (I), worin R¹, R², R³, R⁴, R⁹, R¹⁰, A, D, E die obengenannte Bedeutung haben, B ein Rest der Formel (VIII) und R⁵ ein Rest der Formel IV ist, umsetzt, wobei man zunächst jedoch die Verbindungen der Formel (XVII) oder das Amin oder den Alkohol mit einer doppelt aktivierten Carbonylverbindung zur Bildung der Harnstoff- bzw. Urethangruppe, z.B. mit Carbodiimiden, Phosgen oder Chlorkohlensäureestern, bevorzugt Phosgen und Carbonyldiimidazol reagieren läßt, oder
   f3) eine Verbindung der Formel (XVII) mit einem entsprechenden Isocyanat oder Isothiocyanat, bevorzugt bei einer Temperaturen von 0°C bis Raumtemperatur in inerten Lösungsmitteln, bevorzugt Dichlormethan oder Dimethoxyethan, zu einer Verbindung der Formel (I), worin R¹, R², R³, R⁴, R⁹, R¹⁰, A, D, E die obengenannte Bedeutung haben, B ein Rest der Formel (VIII) und R⁵ ein Rest der Formel IV ist, umsetzt, und
   g) die erhaltenen Verbindungen der Formel (I) gegebenenfalls nach bekannten Methoden in ihre physiologisch verträglichen Salze überführt;
   oder
β.
   a) eine Verbindung der Formel (XI) 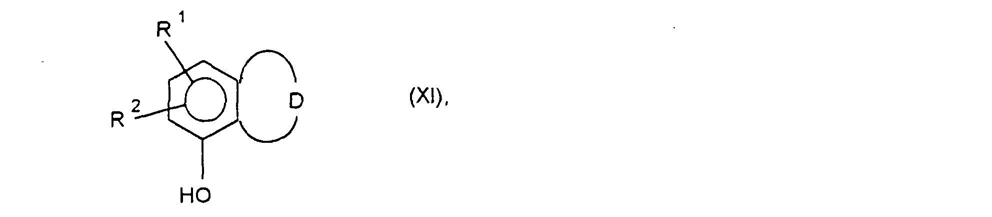 worin R¹, R² und D wie oben definiert sind, in Gegenwart von Metallhydriden, wie Lithium-, Kalium- oder Natriumhydrid, oder Alkalimetallcarbonaten, wie Na₂CO₃, K₂CO₃ oder Cs₂CO₃ in einem inerten Lösungsmittel, wie DMF oder DMSO, bei einer Temperatur von 0°C bis 60°C, bevorzugt bei 20 bis 30°C, mit einer Verbindung der Formel (XVIII) 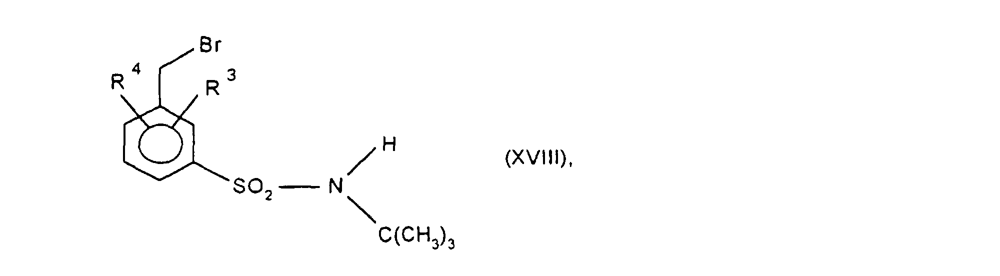 worin R³ und R⁴ wie oben in Formel (I) definiert sind, umsetzt;
   b) die aus dieser Verbindung resultierende Verbindung der Formel (XIX) 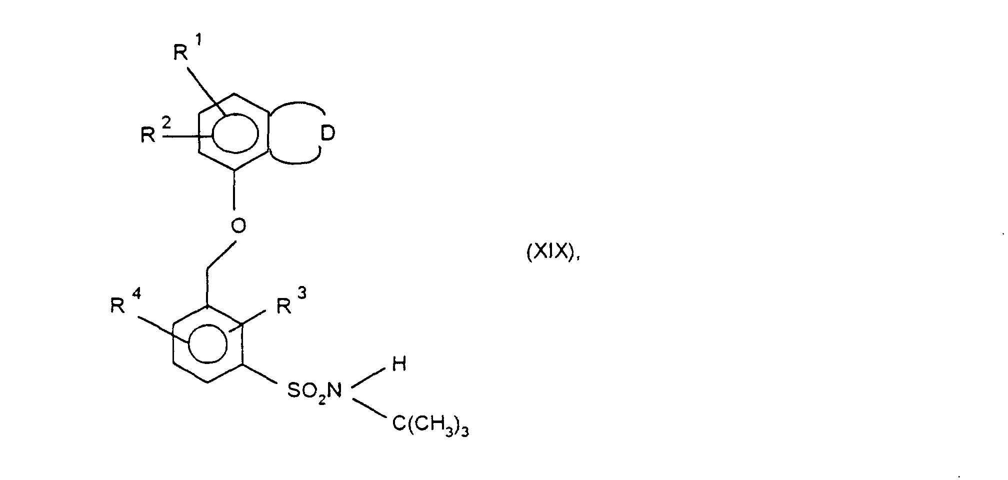 worin R¹, R², R³, R⁴ und D wie oben in Formel (I) definiert sind, in Gegenwart von Metallhydriden wie Natriumhydrid in inerten Lösungsmitteln wie DMF, THF oder DMSO mit Alkyl- oder Arylhalogeniden R⁹-Hal, wobei R⁹ für Alkyl und Aryl wie oben definiert steht und Hal vorzugsweise Iodid ist, bei einer Temperatur von 0°C bis 40°C, umsetzt;
   c) die resultierende Verbindung der Formel (XX) 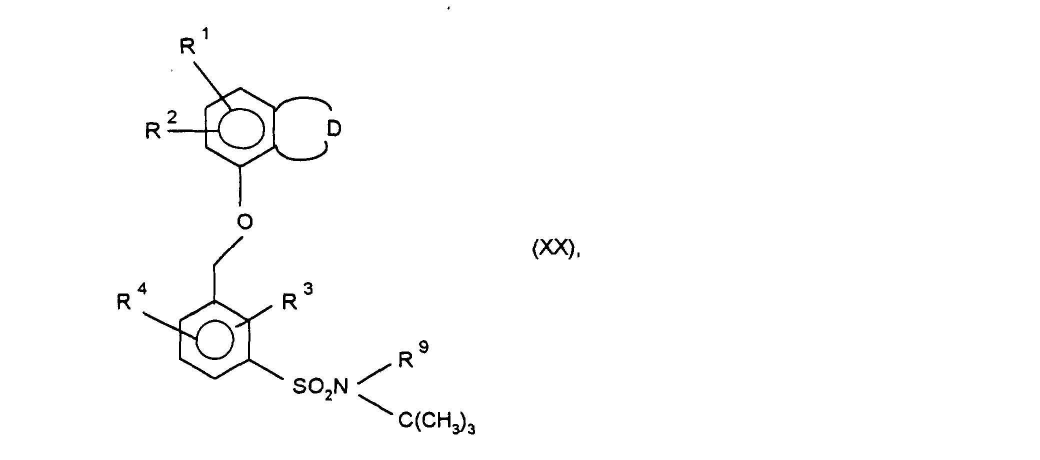 worin R¹, R², R³, R⁴, R⁹ und D wie oben in Formel (I) definiert sind, zunächst mit einem Überschuß an Säure, bevorzugt Trifluoressigsäure in Gegenwart eines Kationfängers wie Anisol für 4 bis 24 h bei einer Temperatur von 20°C bis 60°C in einem inerten Lösungsmittel wie CH₂Cl₂ behandelt und die erhaltene Verbindung anschließend in Gegenwart einer anorganischen oder organischen Base wie z.B. Cs₂CO₃ oder NaH, mit Halogeniden der Form Hal-R¹⁰, worin R¹⁰ die oben angegebene Bedeutung, ausgenommen Wasserstoff, hat, zu Verbindungen der Formel (I), worin R¹, R², D, R³, R⁴, R⁹ und R¹⁰ die obengenannte Bedeutung haben, B ein Rest der Formel (VIII) und R⁵ ein Rest der Formel (V) ist, umsetzt, und
   d) die so erhaltenen Verbindungen der Formel (I) gegebenenfalls in ihr physiologisch verträgliches Salz überführt.

Verfahren zur Herstellung der Verbindungen der Formel (XI), in welcher D für einen Rest der Formel (VI) steht, sind u.a. bekannt aus H. Fiedler, J. Prakt. Chemie, Bd 13, 1961, 86 ff.

Verfahren zur Herstellung der Verbindungen der Formel (XI), in welcher D für einen Rest der Formel (VII) steht, sind u.a. bekannt aus G. Gribble et al., Synthesis 10, (1975), S. 650-652 und J.M. Schaus et al., Synth. Commun. 20, (1990), 3553-3562.

Als Ausgangsstoff für die Brommethylverbindung der Formel (XII) kann beispielsweise eine käuflich erhältliche halogenierte, nitrierte aromatische Methylverbindung wie 2,6-Dichloro-3-nitro-toluol verwendet werden, die zunächst in das entsprechende Cyano-Derivat überführt wird.
Die Herstellung des Cyano-Derivats erfolgt durch Ersatz von Halogen (z.B. Chlor) gegen Cyano durch Einwirken von Cyaniden, bevorzugt Kupfercyanid, in inerten hochsiedenden Lösungsmitteln wie DMF oder N-Methylpyrrolidon bei deren Siedepunkt.
Die Konversion zu der entsprechenden Brommethylverbindung der Formel (XII) erfolgt durch Umsetzung der Methylgruppe des zuvor erhaltenen Cyano-Derivats mit N-Bromsuccinimid oder 1,3-Dibrom-5,5-dimethylhydantoin in inerten Lösungsmitteln, bevorzugt Chlorbenzol oder Cyclohexan bei Temperaturen von 60°C bis zum Siedepunkt.

Als Ausgangsstoff für die Brommethylverbindung der Formel (XVIII) kann beispielsweise eine käuflich erhältliche halogenierte, sulfonierte aromatische Methylverbindung verwendet werden, deren Sulfochlorid-Derivat mit t-Butylamin in CH₂Cl₂ bei Raumtemperatur umgesetzt wird, wobei die für die nachfolgende Bromierung benötigte Methylverbindung erhalten wird.
Die Konversion zu der Brommethylverbindung der Formel (XVIII) erfolgt durch Umsetzung der Methylgruppe des zuvor erhaltenen Sulfon-Derivats mit N-Bromsuccinimid oder 1,3-Dibrom-5,5-dimethylhydantoin in inerten Lösungsmitteln, bevorzugt Chlorbenzol oder Cyclohexan bei Temperaturen von 60°C bis zum Siedepunkt.

Die weiteren Ausgangsverbindungen können wie folgt hergestellt werden:

Die Alkoxy- oder Thioalkylverbindungen werden im allgemeinen durch Austausch von Halogen (z.B. Chlor) gegen Alkoxy oder S-Alkyl hergestellt. Die Reaktion mit den entsprechenden Alkoholaten oder Thiolaten, bevorzugt deren Alkali- oder Erdkalkalisalzen, erfolgt in inerten Lösungsmitteln zwischen 0°C und 60°C, bevorzugt zwischen 0°C und Raumtemperatur.

Die Herstellung der Cyano-Derivate erfolgt im allgemeinen durch Ersatz von Halogen (z.B. Chlor) gegen Cyano durch Einwirken von Cyaniden, bevorzugt Kupfercyanid, in inerten hochsiedenden Lösungsmitteln wie DMF oder N-Methylpyrrolidon bei deren Siedepunkt.

Die Amid-Verbindungen resultieren im allgemeinen aus den entsprechenden Nitril-Verbindungen durch Behandlung mit alkalischer H₂O₂-Lösung in alkoholischer Lösung bei Temperaturen von Raumtemperatur bis zum Siedepunkt.

Die phenolischen Verbindungen resultieren im allgemeinen aus der Behandlung der entsprechenden Alkoxyderivate mit Lewis-Säuren wie Bortribromid in inerten Lösungsmitteln bei Temperaturen zwischen 0°C und Raumtemperatur.

Als aktivierte Säurederivate werden bei Schritt α.f1) Säurechloride, Säureanhydride und Aktivester verwendet, z.B. Carbon- und Sulfonsäurechloride und -bromide, gemischte Anhydride, symmetrische Anhydride, p-Nitrophenylester und Hydroxysuccinimidester. Die Wahl eines dieser aktivierten Derivate ist abhängig von der einzuführenden Acyl- bzw. Sulfonylgruppe. Im Falle der freien Carbonsäuren erfolgt die Umsetzung in Gegenwart von in der Peptidchemie angewandten Kondensationsreagenzien, siehe z.B. Houben-Weyl, Methoden der Organischen Chemie, Bd 15/2, Georg Thieme Verlag, Stuttgart, 1974, insbesondere Carbodiimide wie N,N'-Dicyclohexylcarbodiimid, N,N'-Diisopropylcarbodiimid und N-Ethyl-N'-(3-dimethylaminopropyl)-carbodiimid oder Chromiumsalze wie O-[Cyan-(ethoxycarbonyl)methylenamino]-1,1,3,3-tetramethyluruniumtetrafluoroborat (TOTU) und O-Benzotriazol-1-yl-N,N,N',N'-tetramethyluronium-hexafluorophosphat (MBTU).

Die erfindungsgemäßen Verbindungen der Formel (I) haben einzeln oder in Kombination eine Bradykinin-antagonistische Wirkung, die in verschiedenen Modellen getestet werden kann (siehe Handbook of Exp. Pharmacol. Vol. 25, Springer Verlag, 1970, S. 53-55), so z.B. am isolierten Rattenuterus, am Meerschweinchenileum, an der isolierten Pulmonalarterie des Meerschweinchens oder an der Jugularvene des Kaninchens.

Die Effekte der Verbindungen der Formel (I) auf die Bradykinin-induzierte Bronchokonstriktion und auf das Carregeenin-induzierte Pfotenödem können analog zu der in der Br. J. Pharmacol., 102, 774-777 (1991) beschriebenen Vorgehensweise bestimmt werden.

Der natriuretische und diuretische Effekt der Verbindungen der Formel (I), einschließlich der unter β. davon ausgenommenen Verbindungen, bei chronischen fibrogenetischen Lebererkrankungen und akuten Lebererkrankungen kann im Modell der CCl₄-induzierten Leberfibrose an der Ratte (Bickel et al., J. Hepatol. (1991), 13 (Suppl. 3), S26-S33) bestimmt werden.

Die Wirkung der Verbindungen der Formel (I), einschließlich der unter β. davon ausgenommenen Verbindungen auf die durch das Alzheimer-Protein Amyloid (β/A4) stimulierte c-GMP-Produktion in isolierten Endothelzellen kann folgendermaßen getestet werden:

### Testsysteme:

Bovine Aorten-Endothelzell-Kulturen und mikrovaskuläre koronare Endothelzell-Kulturen

### Methode:

Bestimmung des Einflusses von Bradykinin-Rezeptorantagonisten der Formel (I) auf die durch Gabe von 1 µmol/l des Alzheimer-Proteins β/A4 stimulierte Produktion von cGMP in Endothelzellkulturen.
cGMP: zyklisches Guanosinmonophosphat

Es ist hinreichend gezeigt worden, daß Endothelzellen ein geeignetes Testsystem zum Nachweis einer Wirkung und Freisetzung von Bradykinin darstellen (G. Wiemer et al., Hypertension 1991; 18: 558-563). An Endothelzellen führt Bradykinin zur Erhöhung der Produktion von cGMP, welches mittels eines Radioimmunassays bestimmt wird. Erhöhung der Bildung von cGMP durch Bradykinin ist ein Indikator für eine Freisetzung von NO (Stickstoffmonoxid) aus Endothelzellen.

### Ergebnis:

Die gleichzeitige Inkubation der o.a. Zellkulturen mit den Verbindungen der Formel (I), einschließlich der unter β. davon ausgenommenen Verbindungen, in Konzentrationen von 10 nM/l bis 10 µM/l verhindert die vom β/A4-Protein ausgelöste Stimulierung der Produktion von cGMP.

### Bewertung

Der durchgeführte Versuch zeigt an, daß die Wirkung des Alzheimer-Proteins β/A4 auf die Produktion von cGMP über eine Bindung von Bradykinin an seine Zellrezeptoren vermittelt ist.

Endothelzellkulturen dienen hierbei als Indikator einer Wirkung von β/A4, die über Bradykinin vermittelt ist. Die Endothelzellen sind dabei jedoch nicht nur das Indikatorsystem für eine Wirkung über Bradykinin-Rezeptoren, sondern auch das Effektororgan bei der Alzheimer'schen Krankheit. Endothelzellen sind Bestandteile der Blutgefäße und bilden diese aus. Die Blutgefäße selbst sind von Ablagerungen des Alzheimer-Proteins Amyloid (β/A4) bei der Alzheimer'schen Krankheit neben neuronalem Gewebe stark betroffen. Endothelzellen sind verantwortlich für eine durch Bradykinin ausgelöste Steigerung der Durchlässigkeit der Bluthirnschranke.

Die Bestimmung der Affinität der Verbindungen der Formel (I) zum Bradykinin B₂ Rezeptor erfolgte an Membranpräparationen des Meerschweinchenileums (R.B. Innis et al., Proc. Natl. Acad. Sci. USA; 17 (1981) 2630) nach folgendem Verfahren:

### 1. Ligand: ³H-BRADYKININ (von NEN Du Pont)

### 2. Pufferansätze:

a) TES-Puffer:
   25 mM TES (SIGMA, Best.Nr.: T-4152)
   1 mM 1,10-Phenanthrolin (SIGMA; Best.Nr.: P-9375)
b) Inkubations-Puffer:
   25 mM TES (SIGMA; Best.Nr.: T-4152)
   1 mM1, 10-Phenanthrolin (SIGMA; Best.Nr.: P-9375)
   0,1 % Albumin, Bovine (SIGMA; Best.Nr.: A-7906)
   140 µg/ml Bacitracin (SIGMA; Best.Nr.: B-0125)
   1 mM Dithiothreitol (SIGMA; Best.Nr.: D-0632)
   1 µM Captopril - 1-[(2S)-3-Mercapto-2-methylpropionyl]-L-prolin
   Beide Puffer werden mit 5 molarer NaOH auf pH 6,8 eingestellt.

### 3. Membranpräparation:

Meerschweinchen-Ilea werden durch vorsichtiges Ausstreichen grob vom Darm-Inhalt befreit und in 0,9%iger NaCl-Lösung gesäubert.

Die ca. 2 cm langen Ilea-Stücke werden in eiskalten TES-Puffer überführt (ca. 1 g /10 ml) und mit dem Ultraturrax ca. 30 sec. lang im Eisbad homogenisiert. Das Homogenat wird anschließend durch 3 Lagen Gaze gefiltert und das Filtrat bei 50.000 g/10 Minuten zentrifugiert.

Der Überstand wird verworfen, das Pellet in dem gleichem Volumen TES-Puffer rehomogenisiert und erneut bei 50.000 g/10 Minuten zentrifugiert. Das Pellet wird in Inkubations-Puffer rehomogenisiert (ca. 1 g/5 ml) und in Kryoröhrchen, 2 ml portioniert, bei -70°C eingefroren.

Die Proteinkonzentration der fertigen Membransuspension wird nach LOWRY bestimmt und sollte ca. 15 µg /100 µl betragen.

### 4. Bindungstest:

Alle Inkubationen werden bei Raumtemperatur für 60 Minuten auf Mikrotiterplatten (96 x 300 µl) in 200 µl Volumen durchgeführt. Alle Ansätze in Inkubations-Puffer. Dazu werden 50 µl des Radioliganden, 50 µl des zu prüfenden Präparats und 100 µl der Membransuspension nacheinander in die Vertiefungen der Mikrotiterplatte pipettiert.

### a) Sättigungsexperimente (Heiße Sättigung):

Herstellung der ³H-Bradykinin-Lösung: Für die Sättigungsexperimente werden die Konzentrationen 0.05, 0.1, 0.2, 0.4, 0.6, 0.8, 1.0, 1.5, 2.0, 2.5 und 3.0 nMol/l, das entspricht 0.05 bis 3.0 pMol/ml, eingesetzt. Nach der Herstellung der entsprechenden Verdünnungen werden je 50 µl pro Probe vorgelegt.

Unspezifische Bindung: Für jede Konzentration des radioaktiven Liganden muß die unspezifische Bindung bestimmt werden. Dies kann durch Zugabe einer hohen Konzentration (1 -100 µMol) des nichtmarkierten Liganden, anderer Antagonisten oder Agonisten des Bradykinin-Rezeptors erreicht werden. In diesem Test wird HOE 140 (10 µMol/l) verwendet. Dazu werden 1,862 mg in 1 ml Dimethylsulfoxid (DMSO) gelöst, 1:25 mit Inkubationspuffer verdünnt und von dieser Lösung 50 µl zu den Proben in die Mikrotiterplatte gegeben. Die Reaktion wird durch die Zugabe von 100 µl der Membransuspension gestartet.

### b) Kompetitionsexperimente (IC₅₀):

Hier werden eine feste Größe des radioaktiven Liganden (0,25 bis 0,3 nMol/l ³H-Bradykinin) und verschiedene Konzentrationen der nichtmarkierten Agonisten oder Antagonisten eingesetzt.

Zu jeweils 50 µl der ³H-Bradykinin-Lösung werden 50 µl der zu prüfenden Präparate bzw. Standards in den Konzentrationen 10⁻⁵ bis 10⁻¹⁰ Mol/l zugegeben und die Reaktion durch Zugabe von 100 µl Membransuspension gestartet. Auch in diesem Test werden 3fach-Bestimmungen durchgeführt und drei Proben zur Bestimmung der unspezifischen Bindung mit 10 µMol/l HOE 140 inkubiert.

Die auf Kompetition zu prüfenden Präparate werden grundsätzlich in einer Konzentration von 1 mMol/l in Dimethylsulfoxid (DMSO) gelöst, und anschließend mit DMSO weiterverdünnt. Diese Lösung wird dann 1:25 mit Inkubationspuffer verdünnt.

Nach der Inkubation werden die Proben im Skatron Zellharvester über einen vorher mit 0, 1 % PEI (Polyethylenimin) angefeuchteten Whatmann GF/B Filterpapierstreifen abfiltriert und mit, je Probe, 10 ml eiskaltem TES-Puffer nachgewaschen. Die noch feuchten Filter werden in Mini-Scintillationsröhrchen ausgestanzt und mit 3 ml Scintillator aufgefüllt.

Nach ca. 12 Stunden Einweichzeit werden die Proben kurz aufgeschüttelt und im Beta-Counter gemessen.

### c) Screening:

Im Primär-Screening werden im allgemeinen nur 1-2 Konzentrationen des Prüfpräparats (10⁻⁵ und 10⁻⁶ Mol/l) eingesetzt. Ist bei der höchsten Konzentration eine Verdrängung des Radioliganden von 50% oder mehr nachweisbar, wird eine vollständige Analyse (Kompetitions-Experiment) mit mind. 8 Konzentrationen vorgenommen.

### 4. Auswertung:

Die Auswertung erfolgt mittels des LIGAND-Programmpakets (Mc Pherrson, Minson & Rodbard, Vertrieb: Elsevier-BIOSOFT), das die notwendigen Berechnungen zur Bestimmung von IC₅₀ und Kᵢ-Werten vornimmt. Dieses Programm führt außerdem grafische Darstellungen der Sättigungs- bzw. Verdrängungs-Kurven sowie den SCATCHARD-Plot, HILL-Plot oder HOFSTEE-Plot durch.

### 5. Testergebnisse

Nach dem oben angeführten Verfahren wurden für die Verbindungen der Beispiele 1, 6, 9, 16, 20 und 24 als repräsentative Verbindungen der beschriebenen Benzyloxysubstituierten, anellierten N-Heterocyclen der Formel (I) folgende Kᵢ-Werte bestimmt:

| Beispiel | Kᵢ [nM] |
|---|---|
| 1 | 8.3 |
| 6 | 0.1 |
| 9 | 6.0 |
| 16 | 17.4 |
| 20 | 5.4 |
| 24 | 1.0 |

Desweiteren kann zur Bestimmung der Bradykinin-antagonistischen Wirkung der Verbindungen der Formel (I) ihr Effekt auf die bradykinin-induzierte Kontraktion des Meerschweinchen-Ileums nach folgendem Protokoll gemessen werden:

Meerschweinchen im Gewicht von ca. 300 g (Morioth strain, ♂♀) werden durch Nackenschlag getötet und entblutet. Das Ileum wird in einer Länge von ca. 20 cm herauspräpariert, mit Tyrode-Lösung durchspült (Recordspritze) und so vom Darminhalt befreit. Nun wird es in 1,5 cm lange Abschnitte geteilt. Diese werden im 10 ml fassenden Organbädern, die mit Tyrode-Lösung gefüllt sind, fixiert und mit Dehnungsmeßstreifen (isometrische Kontraktionsmessung) verbunden. Die Vorlast beträgt 1 g. Die Tyrode-Lösung wird in einem Wasserbad auf 37°C erwärmt und mit Druckluft durchperlt.
Nach einem Intervall von 30 Min. wird mit dem Versuch begonnen. Nach Aufzeichnung der biologischen Null-Linie wird pro Organbad Bradykinin in einer Endkonzentration von 4 x 10⁻⁸ mol/l zugesetzt und die Konzentration aufgezeichnet. Danach wird 3 Min. mit Tyrode-Lösung gespült und nach einer Ruhepause von 20 Min. wieder Bradykinin hinzugefügt. Das Maximum der Kontraktion ist erreicht (Kontrolle). Wieder spülen, Ruhepause. Nun wird der Bradykinin-Antagonist zugefügt (Einwirkungszeit 10 Min.). Danach wird wieder Bradykinin zugesetzt und die nun erfolgende Kontraktion mit der der Kontrolle verglichen. Die Aufzeichnung des Versuchs erfolgt auf einem Tintenschreiber (Goerz Metrawatt SE 460, BBC).

| | | |
|---|---|---|
| Tyrode-Lösung (mM) | NaCl | 137 |
| | Glucose | 5,05 |
| | KCI | 2,68 |
| | NaHCO₃ | 11,9 |
| | NaH₂PO₄ | 0,47 |
| | MgCl₂ x 2H₂O | 0,49 |
| | CaCl₂ x 2H₂O | 0,68 |

- Verstärker:: TF6 V3 Fa. Fleck, Mainz
- Bradykinin:: Fa. Bachem

So besitzen z.B. die Verbindungen der Beispiele 6 und 20 folgende, nach obigem Verfahren ermittelte IC₅₀-Werte:

| Beispiel | IC₅₀ |
|---|---|
| 6 | 3.5 x 10⁻⁷ M |
| 20 | 5.6 x 10⁻⁸ M |

Für die orale Anwendungsform oder zur Applikation auf die Schleimhäute werden die aktiven Verbindungen mit den dafür üblichen Zusatzstoffen wie Trägerstoffen, Stabilisatoren oder inerten Verdünnungsmittein vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesia, Magnesiumcarbonat, Kaliumphosphat, Milchzucker, Glucose, Magnesiumstearylfumarat oder Stärke, insbesondere Maisstärke verwendet werden. Dabei kann die Zubereitung sowohl als Trockenund Feuchtgranulat erfolgen. Als ölige Trägerstoffe oder Lösungsmittel kommen beispielsweise pflanzliche oder tierische Öle in Betracht, wie Sonnenblumenöl und Lebertran.

Ein Präparat für die topische Anwendung kann als wäßrige oder ölige Lösung, Lotion, Emulsion oder Gelee, Salbe oder Fettsalbe oder, falls möglich, in Sprayform vorliegen, wobei gegebenenfalls durch Zusatz eines Polymers die Haftung verbessert werden kann.

Für die intranasale Anwendungsform werden die Verbindungen mit den dafür üblichen Zusatzstoffen wie Stabilisatoren oder inerten Verdünnungsmitteln vermischt und durch übliche Methoden in geeignete Darreichungsformen gebracht, wie wäßrige, alkoholische oder ölige Suspensionen oder wäßrige, alkoholische oder ölige Lösungen. Wäßrigen intranasalen Zubereitungen können Chelatbildner, Ethylendiamin-N,N,N',N'-tetraessigsäure, Citronensäure, Weinsäure oder deren Salze zugefügt werden. Die Applikation der Nasallösungen kann mittels Dosierzerstäuber erfolgen oder als Nasaltropfen mit viskositätserhöhendem Anteil bzw. Nasengels oder Nasencremes.

Die beschriebenen Verbindungen der Formel (I) und deren pharmakologisch geeigneten Salze sind potente Bradykinin-Antagonisten. Daher können sie zur Behandlung und/oder der Prävention aller pathologischer Zustände, die durch Bradykinin und Bradykinin-analoge Peptide vermittelt, ausgelöst oder unterstützt werden, verwendet werden. Dies beinhaltet u.a. Allergien, Entzündungen, Autoimmunerkrankungen, Schock, Schmerz und, mehr speziell, Asthma, Husten, Bronchitis, Rhinitis, chronisch obstruktive Pulmonalerkrankungen, Pneumonitis, septischen Schock, endotoxischen Schock, anaphylaktischen Schock, disseminierenden intravaskulären Koagulopathie, Arthritis, Rheuma, Osteoarthritis, Lumbago, entzündungsinduzierte Knochenresorption, Konjunctivitis, Iritis, Kopfschmerz, Migräne, Zahnschmerz, Rückenschmerz, krebsbedingte Schmerzen, postoperativen Schmerz, Traumata (Wunden, Verbrennungen usw.), Ausschlag, Erytheme, Ödeme, Ekzeme, Dermatitis, Zoster, Herpes, Juckreiz, Psoriasis, Flechte, entzündliche Darmerkrankungen, Hepatitis, Pankreatitis, Gastritis, Ösophagitis, Nahrungsallergien, Geschwüre, Reizdarm, Angina, Hirnödem, niedriger Blutdruck, Thrombose, Schädel-Hirnund Wirbelsäulen-Trauma, Frühgeburt, Atherosklerose, Aszites bei Malignom, Tumormetastasen, Hirnödem bei Tumoren Hitzeschädigung des Gehirns, Virus-Erkrankungen, Leberzirrhose und Alzheimer'sche Krankheit.

Die von den Verbindungen der Formel (I) unter β. ausgenommenen Verbindungen der Formel I, können ebenfalls zur Behandlung und/oder Prävention der Leberzirrhose und/oder Alzheimer'schen Krankheit verwendet werden.

Da weiterhin bekannt ist, daß Bradykinin verknüpft ist mit der Freisetzung von Mediatoren, wie Prostaglandinen, Leukotrienen, Tachykininen, Histamin, Thromboxanen, besitzen die Verbindungen der Formel (I) somit auch das Potential zur Behandlung und/oder Prävention von den Krankheiten, die durch diese Mediatoren hervorgerufen werden.

Die Erfindung betrifft daher auch die Verwendung von Verbindungen der Formel (I) als Heilmittel und pharmazeutische Präparate, die diese Verbindungen enthalten.

Pharmazeutische Präparate und Heilmittel enthalten eine wirksame Menge des Wirkstoffs der Formel (I) - einzeln oder in Kombination - zusammen mit einem anorganischen oder organischen pharmazeutisch verwendbaren Trägerstoff.

Die Anwendung kann enteral, parenteral - wie z. B. subkutan, i.m. oder i.v. -, sublingual, epikutan, nasal, rektal, intravaginal, intrabukkal oder per Inhalation erfolgen. Die Dosierung des Wirkstoffs hängt von der Warmblüter-Spezies, dem Körpergewicht, Alter und von der Applikationsart ab.

Die pharmazeutischen Präparate der vorliegenden Erfindung werden in an sich bekannten Lösungs-, Misch-, Granulier- oder Dragierverfahren hergestellt.

Für die inhalative Anwendung können Vernebler oder Druckgaspackungen unter Verwendung inerter Trägergase benutzt werden.

Zur intravenösen, subkutanen, epikutanen oder intradermalen Applikation werden die aktiven Verbindungen oder deren physiologisch verträgliche Salze, gewünschtenfalls mit den pharmazeutisch üblichen Hilfsstoffen, beispielsweise zur Isotonierung oder pH-Einstellung sowie Lösungsvermittler, Emulgatoren, oder anderen Hilfsstoffen, in Lösung, Suspension oder Emulsion gebracht.

Sollten die Halbwertszeiten der beschriebenen Arzneistoffe in Körperflüssigkeiten unzureichend sein, ist der Einsatz von injizierbaren Retardzubereitungen sinnvoll.

Als Arzneiformen können z.B. ölige Kristallsuspensionen, Mikrokapseln, Rods oder Implantate verwendet werden, wobei die letzteren aus gewebsverträglichen Polymeren, insbesondere bioabbaubaren Polymeren, wie z.B. auf der Basis von Polymilchsäure-Polyglykolsäure-Copolymeren oder Humanalbumin aufgebaut sein können.

Ein geeigneter Dosisbereich für topische und inhalative Anwendungsformen sind Lösungen mit 0,01-5 mg/l, bei systemischen Applikationsformen sind 0,01-10 mg/kg geeignet.

Allgemein können Mengen zwischen 0,1 bis 1000 mg/kg Körpergewicht appliziert werden.

### Liste der Abkürzungen:

- abs.: absolut
- BOC: t-Butyloxycarbonyl
- CH₂Cl₂: Dichlormethan
- DCI: Desorption Chemical Ionisation
- DMAP: Dimethylaminopyridin
- DMF: N,N-Dimethylformamid
- EE: Ethylacetat
- ESI: electron spray ionisation
- FAB: Fast Atom Bombardment
- Fp: Schmelzpunkt
- ges.: gesättigt
- h: Stunde(n)
- Hal: Halogen
- MeOH: Methanol
- Min: Minute(n)
- RT: Raumtemperatur
- TOTU: O-[Cyan(ethoxycarbonyl)methylenamino)-1,1,3,3-tetramethyluronium-tetrafluoroborat
- Zers.: Zersetzung

Die Erfindung wird durch nachstehende Beispiele erläutert.

### Beispiel 1

### 8-[6-chloro-2-cyano-3-(N-ethylaminocarbonylglycyl-N-methyl)amino-benzyloxy]-2-methylchinolin

### a) 3-Chloro-2-methyl-6-nitro-benzonitril

Eine Lösung von 10 g (49 mmol) 2,6-Dichloro-3-nitro-toluol und 4,8 g (54,0 mmol) CuCN in 100 ml abs. DMF wurde 6h bei 150°C gerührt, wobei nach 4h weitere 2,4 g (27,0 mmol) CuCN hinzugefügt wurden. Das Lösungsmittel wurde abgezogen und der erhaltene Rückstand in Essigester aufgenommen. Der ausgefallene Niederschlag wurde abfiltriert und mehrfach mit warmem Essigester extrahiert. Die vereinigten Essigester-Lösungen wurden mit H₂O, verdünnter Ammoniak- und gesättigter NaCl-Lösung gewaschen, über Na₂SO₄ getrocknet und zur Trockene eingeengt. Der resultierende gelbe, kristalline Rückstand wurde säulenchromatograhisch an SiO₂ mit EE/n-Neptan 1:10 als Lösungsmittel gereinigt. Es resultierten 5,8 g der Titelverbindung in Form gelber Kristalle.
Fp: 93-96°C
R_{f} (SiO₂, EE/n-Heptan 1:2) = 0,39
MS(DCI): 197 (M+H)

### b) 6-Chloro-2-cyano-3-nitro-benzylbromid

Eine Suspension von 5,0 g (25,4 mmol) der Verbindung aus Beispiel 1a) und 8,0 g (28,0 mmol) 1,3-Dibrom-5,5-dimethylhydantoin in 100 ml Chlorbenzol wurden nach Zugabe von 300 mg Benzoylperoxid 18h bei 110°C gerührt. Die Reaktionslösung wurde im Hochvakuum zur Trockne eingeengt, und der kristalline, braune Rückstand in CH₂Cl₂ aufgenommen. Die CH₂Cl₂-Lösung wurde mit H₂O, 5%iger Na₂SO₃-Lösung, H₂O, 10 %iger NaHCO₃-Lösung und schließlich gesättigter NaCl-Lösung gewaschen. Trocknung, Einengung und säulenchromatographische Reinigung des Rückstandes an SiO₂ (EE/n-Heptan 1:4) lieferte 3,5 g der Titelverbindung als beige Kristalle.
Fp: 89°C
R_{f} (SiO₂, EE/n-Heptan 1:2) = 0,28
MS(DCI): 275/277 (M+H)

### c) 8-(6-Chloro-2-cyano-3-nitro-benzyloxy)-2-methylchinolin

Unter einer Argon-Atmosphäre wurde eine Suspension von 1,4 g (9,0 mmol) 8-Hydroxychinaldin und 2,93 g (9,0 mmol) Cs₂CO₃ in 15 ml abs. DMF für 30 Min bei RT gerührt. Hierzu wurde eine Lösung von 2,5 g (9,0 mmol) der Verbindung aus Beispiel 1 b) in 15 ml abs. DMF getropft. Die resultierende gelbe Suspension wurde 3h bei RT gerührt. Die Reaktionslösung wurde im Hochvakuum zur Trockene eingeengt, der Rückstand in CH₂Cl₂ aufgenommen und nacheinander mit H₂O, 10 % NaHCO₃-, 5 % NaHSO₄ und ges. NaCl-Lösung gewaschen. Trocknung über MgSO₄, Einengung und Umkristallisation aus EE lieferte 2,4 g der Titelverbindung in Form gelber Kristalle.
Fp: 212-215°C
R_{f} (SiO₂, EE/n-Heptan 1:2) = 0,11
MS(DCI): 354 (M+H)

### d) 8-(3-Amino-6-chloro-2-cyano-benzyloxy)-2-methylchinolin

2,0 g (5,5 mmol) der Verbindung aus Beispiel 1c) wurde in 60 ml EE gelöst und mit 6,2 g (27,5 mmol) SnCl₂ x 2H₂O versetzt. Die resultierende Suspension wurde für 40 Min unter Rückfluß gerührt.Es wurde zur Trockene eingeengt, der Rückstand in H₂O aufgenommen und durch Zusatz von 2N NaOH der pH-Wert auf 9 eingestellt.

Der ausfallende Niederschlag wurde abgesaugt und mehrfach mit warmem CH₂Cl₂ ausgerührt. Einengung der CH₂Cl₂-Lösung ergab 1,4 g der Titelverbindung in Form eines hellbraunen Feststoffes.
Fp: 248-251 °C
R_{f} (SiO₂, EE/n-Heptan) = 0,13
MS(DCI): 324 (M+H)

### e) 8-[6-Chloro-2-cyano-3-(N-phthaloylglycyl)amino-benzyloxy]-2-methylchinolin

Eine Lösung aus 1,4 g (4,4 mmol) der Verbindung aus Beispiel 1d), 540,0 mg (4,4 mmol) DMAP, 355 µl (4,4 mmol) Pyridin und 2,2 g (6,6 mmol) Phthalimidoacetylchlorid in 100 ml CH₂Cl₂ wurde 45 Min unter Rückfluß gerührt. Die Reaktionslösung wurde mit H₂O gewaschen, über Na₂SO₄ getrocknet und eingeengt. Der erhaltene Rückstand wurde durch Umkristallisation aus Methylenchlorid gereinigt. Es resultierten 1,2 g der Titelverbindung.
Fp: 209°C
R_{f} (SiO₂, CH₂Cl₂/MeOH 10:1) = 0,72
MS(DCl): 511 (M+H)

### f) 8-[6-Chlor-2-cyano-3-(N-phthaloylglycyl-N-methyl)amino-benzyloxy]-2-methylchinolin

1,2 g (2,3 mmol) der Verbindung aus Beispiel 1e) wurden zu einer auf 0°C gekühlten Suspension aus 113,0 mg (2,3 mmol) NaH (50 %ige Suspension in Öl) in 15 ml abs. DMF hinzugefügt. Es wurde 30 Min bei 0°C gerührt. Anschließend wurden 163 µl (2,6 mmol) Methyliodid zugetropft und die Reaktionslösung für 4h bei 50°C gerührt. Es wurde gekühlt und die Reaktion durch Zugabe von 30 ml H₂O beendet. Es wurden 200 ml CH₂Cl₂ zugefügt, mit H₂O gewaschen, über Na₂SO₄ getrocknet und zur Trockene eingeengt. Säulenchromatographie an SiO₂ des Rückstandes mit CH₂Cl₂/MeOH 20:1 als Laufmittel lieferte 1,0 g der Titelverbindung als amorphen Feststoff.
R_{f} (SiO₂, EE/n-Heptan 1:1) = 0,12
MS(FAB): 525 (M+H)

### g) 8-[6-Chloro-2-cyano-3-(N-glycyl-N-methyl)amino-benzyloxy]-2-methylchinolin

Eine Lösung aus 850,0 (1,6 mmol) der Verbindung aus Beispiel 1f) und 160 µl (3,2 mmol) Hydrazinmonohydrat in 20 ml CH₂Cl₂/Methanol (3:1) wurde für 2h unter Rückfluß gerührt. Anschließend wurde die Reaktionslösung in Wasser suspendiert, der pH auf 12 durch Zugabe von 2N NaOH eingestellt und 3x mit CH₂Cl₂ extrahiert. Die vereinten CH₂Cl₂-Extrakte wurden über Na₂SO₄ getrocknet, eingeengt und der verbleibende Rückstand durch Säulenchromatographie an SiO₂ (CH₂Cl₂/MeOH/NH₄OH 10:1:0,1) gereinigt. Es resultierten 360,0 mg der Titelverbindung als amorpher Feststoff.
R_{f} (SiO₂, CH₂Cl₂/MeOH/NH₄OH 10:1:0,1) = 0,23
MS(DCI): 395 (M+H)

### h) 8-[6-Chloro-2-cyano-3-(N-ethylaminocarbonylglycyl-N-methyl)amino-benzyloxy]-2-methylchinolin

Eine Lösung von 130,0 mg (0,3 mmol) der Verbindung aus Beispiel 1g) und 52 µl (0,6 mmol) Ethylisocyanat wurden für 45 Min unter Rückfluß gerührt. Es wurde zur Trockene eingeengt, der Rückstand mit EE ausgerührt und die Titelverbindung als weißer, amorpher Feststoff abfiltriert. Trocknung im Hochvakuum ergab eine Ausbeute von 66,0 mg der Titelverbindung.
R_{f} (SiO₂, CH₂Cl₂/MeOH/NH₄OH 10:1:0,1) = 0,32
MS(DCI): 466 (M+H)

### Beispiel 2

### 8-[6-Chloro-2-cyano-3-[N-(4-trans-trifluoromethylcinnamoylglycyl)-N-methyl]aminobenzyloxy]-2-methylchinolin

### a) trans-4-Trifluormethylzimtsäurechlorid

200,0 mg (0,92 mmol) trans-4-Trifluormethylzimtsäure wurden mit 200 µl Thionylchlorid versetzt. Nach Zugabe von 2 Tropfen DMF wurde die erhaltene Reaktionslösung für 3h bei 75°C gerührt. Anschließend wurde zur Trockene eingeengt, der Rückstand 2x in Toluol aufgenommen und erneut zur Trockene eingeengt. Trocknung im Hochvakuum lieferte 237,0 mg der Titelverbindung als schwach gelbes, amorphes Pulver.

### b) 8-[6-Chloro-2-cyano-3-[N-(4-trans-trifluoromethylcinnamoylglycyl)-N-methyl]amino-benzyloxy]-2-methylchinolin

Eine Lösung von 100,0 mg (0,253 mmol) der Verbindung aus Beispiel 1 g), 119,0 mg (0,506 mmol) der Verbindung aus Beispiel 2a) und 35µl (0,253 mmol) Triethylamin in 4 ml CH₂Cl₂ wurde 1h unter Rückfluß gerührt. Die Reaktionslösung wurde mit Wasser und gesättigter NaCl-Lösung gewaschen, über Na₂SO₄ getrocknet und eingeengt. Der resultierende ölige Rückstand wurde mittels Säulenchromatographie an SiO₂ (CH₂Cl₂/MeOH 40:1) gereinigt. Es wurden 102 mg der Titelverbindung als amorphes Pulver isoliert.
R_{f} (SiO₂, CH₂Cl₂/MeOH/ 20:1) = 0,32
MS(FAB): 593 (M+H)⁺

### Beispiel 3

### 8-[6-Chloro-2-cyano-3-[N-(3-methoxycinnamoyl-glycyl-N-methyl]amino-benzyloxy]-2-methylchinolin

### a) trans-3-Methoxyzimtsäurechlorid

Die Herstellung der Titelverbindung erfolgte nach dem in Beispiel 2a) angeführten Verfahren. Es resultierten aus 100,0 mg (0,562mmol) 3-Methoxyzimtsäure 120,0 mg der Titelverbindung als amorpher Feststoff.

### b) 8-[6-Chloro-2-cyano-3-[N-(3-methoxycinnamoyl-glycyl-N-methyl]aminobenzyloxy]-2-methylchinolin

Die Herstellung der Titelverbindung erfolgte durch Umsatz der Verbindung aus Beispiel 1g) und der Verbindung aus Beispiel 3a) nach dem in Beispiel 2b) angeführten Verfahren. Es wurden aus 100,0 mg (0,253 mmol) der Verbindung aus Beispiel 1g) 77,0 mg der Titelverbindung als blaß-gelber Feststoff erhalten.
R_{f} (SiO₂, CH₂Cl₂/MeOH/20:1) = 0,20
MS(FAB): 554 (M+H)⁺

### Beispiel 4

### 8-[6-Chloro-2-cyano-3-[N-(4-methoxycarbonylbutanoyl-glycyl-N-methyl]aminobenzyloxy]-2-methylchinolin

Zu einer Lösung von 180,0 mg (0,456 mmol) der Verbindung aus Beispiel 1g) in 8 ml abs. DMF wurden 57 µl (0,456 mmol) mono-Methylglutarat, 65,0 mg (0,456 mmol) Ethyl-(E)-cyanohydroximinoacetat, 155 µl (0,912 mmol) N-Ethyldiisopropylamin und 150,0 mg (0,456 mmol) TOTU hinzugefügt. Es wurde unter Argon für 6h bei RT gerührt. Die Reaktionslösung wurde mit CH₂Cl₂ verdünnt und anschließend mit einer 10 %igen KHSO₄- und einer gesättigter NaHCO₃-Lösung gewaschen. Trocknung über Na₂SO₄, Einengung und Reinigung des Rückstandes durch Säulenchromatographie an SiO₂ mit CH₂Cl₂/MeOH 40:1 als Laufmittel lieferte 156 mg der Titelverbindung als schwach gelbgefärbten, amorphen Feststoff.
R_{f} (SiO₂, CH₂Cl₂/MeOH/ 20:1) = 0,28
MS(FAB): 523 (M+H)⁺

### Beispiel 5

### 8-[6-Chloro-2-cyano-3-[N-(4-carboxybutanoyl-glycyl-N-methyl]amino-benzyloxy]-2-methylchinolin

Eine Lösung von 99,0 mg (0,189 mmol) der Verbindung aus Beispiel 4 in 3 ml Methanol wurde mit 387 µl (0,378 mmol) 1N NaOH-Lösung versetzt und 14h bei RT gerührt. Die Reaktionslösung wurde zur Trockene eingeengt, der Rückstand in wenig H₂O aufgenommen, der pH der Lösung durch Zugabe von 2N HCl auf 5 eingestellt und der ausfallende Niederschlag abgesaugt. Reinigung durch Säulenchromatographie an SiO₂ (CH₂Cl₂/MeOH/ 10:1) lieferte 45 mg der Titelverbindung als blaßgelben, amorphen Feststoff.
R_{f} (SiO₂, CH₂Cl₂/MeOH/ 10:1) = 0,11
MS(ESi): 509 (M+H), 523 (M+Na+H)⁺

### Beispiel 6

### 8-[6-Chloro-2-cyano-3-[N-(4-aminobutylaminocarbonyl-glycyl-N-methyl]aminobenzyloxy]-2-methylchinolin-bis-trifluoracetat

### a) 8-[6-Chloro-2-cyano-3-[N-(4-tert.-butyloxycarbonyl)-aminobutylamino-carbonylglycyl-N-methyl]amino-benzyloxy]-2-methylchinolin

Unter einer Argon-Atmosphäre wurden 77,5 µl (0,405 mmol) N-BOC-1,4-Diaminobutan in 2 ml abs. DMF gelöst, die Lösung mit 68,9 µl (0,405 mmol) N-Ethyldiisopropylamin und 66,0 mg (0,405 mmol) N,N-Carbonyldiimidazol versetzt und für 3h bei RT gerührt. Anschließend wurden 160 mg (0,405 mmol) der Verbindung aus Beispiel 1g) hinzugefügt und die Reaktionsmischung für 48h bei RT gerührt. Es wurde mit EE verdünnt, mit einer ges. Na₂CO₃- und einer 10 %igen KHSO₄-Lösung gewaschen, über Na₂SO₄ getrocknet und eingeengt. Trocknung im Hochvakuum lieferte 86 mg der Titelverbindung als amorphen, beigen Feststoff.
R_{f} (SiO₂, CH₂Cl₂/MeOH/ 10:1) = 0,33
MS(FAB): 609 (M+H)⁺

### b) 8-[6-Chloro-2-cyano-3-[N-(4-aminobutylaminocarbonyl)-glycyl-N-methyl]aminobenzyloxy]-2-methylchinolin-bistrifluoroacetat

Eine Lösung von 80.0 mg (0.131 mol der Verbindung aus Beispiel 6a) in 4 ml CH₂Cl₂ wurde mit 330 µl Trifluoressigsäure versetzt und 2 h bei RT gerührt und danach zur Trockene eingeengt, der Rückstand 2 x in Toluol aufgenommen und erneut zur Trockene eingeengt. Der kristalline Rückstand wurde mit n-Heptan verrieben, abgesaugt und im Hochvakuum getrocknet. Es resultieren 78 mg der Titelverbindung als amorpher, beiger Schaum.
R_{f} (SiO₂/MeOH/NH₄OH 10:1:0,1) = 0,05
MS (ESI): 509 (M+H)⁺

### Beispiel 7:

### [3-Chloro-6-[(3-ethylureido)-acetyl)-methylamino]-2-(2-methylchinolin-8-yloxymethyl]-benzamid

Eine Lösung von 170.0 mg (0.365 mmol) der Verbindung aus Beispiel 1h) in 2 ml Ethanol wurde mit 770 µl einer 3 N Na₂CO₃-Lösung und 230 µl einer 30 %igen H₂O₂-Lösung versetzt. Die resultierende Reaktionslösung wurde für 17 h bei RT gerührt. Es wurde eingeengt, der Rückstand in H₂O aufgenommen und mit CH₂Cl₂ extrahiert. Die vereinten Extrakte wurden über Na₂SO₄ getrocknet, eingeengt und der Rückstand mittels Chromatographie an SiO₂ (CH₂Cl₂ / MeOH 15:1) gereinigt. Es wurden 87 mg der Titelverbindung als amorpher, weißer Feststoff isoliert.
R_{f} (SiO₂, CH₂Cl₂/MeOH 10:1) = 0.34
MS (ESI) : 484 (M+H)⁺

### Beispiel 8

### [3-Chloro-2-(2-methylchinolin-8-yloxymethyl)-6-(N-methyl-[[3-(trans-4-trifluoromethylphenyl)-acryloylamino]N-acetyl]-amino)-benzamid

Die Titelverbindung wurde aus 80.0 mg (0.134 mmol) der Verbindung des Beispiels 2) nach dem in Beispiel 7) angeführten Verfahren hergestellt. Es wurden dabei 44.0 mg der Titelverbindung als weißer, kristalliner Feststoff isoliert.
Fp: 124°C (Zers.)
R_{f} (SiO₂, CH₂Cl₂/MeOH 10:1) = 0.44
MS (FAB): 611 (M+H)⁺

### Beispiel 9

### N-[2-Cyano-4-methoxy-3-(2-methyl-chinolin-8-yloxymethyl)-phenyl]-2-(3-ethylureido)-N-methyl-acetamid

### a) 3-Methoxy-2-methyl-6-nitro-benzonitril

462 mg (20.14 mmol) Natrium wurden bei RT in 15 ml abs. Methanol gelöst. Diese Lösung wurde innerhalb von 2 h in eine auf 55°C erwärmte Lösung von 3.6 g (18.31 mmol) der Verbindung aus Beispiel 1a) in 45 ml abs. Methanol getropft. Nach 1 h Rühren bei 55°C wurde die Reaktionslösung eingeengt, der Rückstand in H₂O/CH₂Cl₂ aufgenommen und die organische Phase abgetrennt. Die organische Phase wurde mit Wasser gewaschen, über Na₂SO₄ getrocknet und eingeengt. Reinigung des erhaltenen Rückstands mittels Säulenchromatographie an SiO₂ (EE/n-Heptan 1:2) lieferte 2.6 g der Titelverbindung als weißen, kristallinen Feststoff.
Fp.: 128-130°C
R_{f} (SiO₂; EE/n-Heptan 2:1) = 0.40
MS (DCI): 193 (M+H)⁺

### b) 2-Cyano-6-methoxy-3-nitro-benzylbromid

Die Titelverbindung wurde aus 6.0 g (31.24 mmol) der Verbindung des Beispiels 9a) nach dem in Beispiel 1b) angeführten Verfahren hergestellt. Die Ausbeute betrug 7.6 g.
Fp.: 137-138°C
R_{f} (SiO₂, EE/n-Heptan 1:1) = 0.29
MS (DCI): 271/273 (M+H)⁺

### c) 8-(2-Cyano-6-methoxy-3-nitro-benzyloxy)-2-methylchinolin

Die Titelverbindung wurde aus der Verbindung des Beispiel 9b) und 8-Hydroxychinaldin nach dem in Beispiel 1c) angeführten Verfahren hergestellt. Es resultierten dabei aus 6.6 g (24.3 mmol) der Verbindung aus Beispiel 9b) 7,35 g der Titelverbindung als weißer, kristalliner Feststoff.
Fp: 227-230°C
R_{f} (SiO₂, EE/n-Heptan 2:1) = 0,10
MS (DCI) : 350 (M+H)⁺

### d) 8-(3-Amino-2-cyano-6-methoxy-benzyloxy)-2-methylchinolin

Die Herstellung der Titelverbindung aus der Verbindung aus Beispiel 9c) erfolgte nach dem in Beispiel 1d) angeführten Verfahren. Aus 6.15 g (17.58 mmol) der Verbindung aus Beispiel 9c) wurden 3.6 g der Titelverbindung erhalten.
Fp : 188-191 °C
R_{f} (SiO₂, EE/n-Heptan 4:1) = 0.20
MS (DCI) = 320 (M+H)⁺

### e) 8-[2-Cyano-6-methoxy-3-(N-phthaloylglycyl)amino-benzyloxy]-2-methylchinolin

Die Titelverbindung resultierte aus der Umsetzung von der Verbindung aus Beispiel 9d) mit Phthalimidoacetylchlorid nach dem in Beispiel 1e) angeführten Verfahren. Aus 2.6 g (8.14 mmol) der Verbindung aus Beispiel 9d) wurden 3.06 g der Titelverbindung erhalten.
Fp: 102-105°C
R_{f} (SiO₂, CH₂Cl₂ 4:19 = 0.34
MS (ESI) : 507 (M+H)⁺

### f) 8-[2-Cyano-6-methoxy-3-(N-phthaloylglycyl-N-methyl)-amino-benzyloxy]-2-methylchinolin

Die Titelverbindung wurde aus der Verbindung aus Beispiel 9e) nach dem in Beispiel 1f) angeführten Verfahren hergestellt. Es resultierten aus 2.4 g (9.60 mmol) der Verbindung aus Beispiel 9e) 2.2 g der gewünschten Verbindung als amorphes Pulver.
R_{f} (SiO₂, CH₂Cl₂/EE 4:1) = 0.10
MS (ESI): 521 (M+H)⁺

### g) 8-[2-Cyano-6-methoxy-3-(N-glycyl-N-methyl)amino-benzyloxy]-2-methylchinolin

Die Herstellung der Titelverbindung aus der Verbindung des Beispiels 9f) erfolgte analog dem in Beispiel 1g) angeführten Verfahren. Aus 630.0 mg (1.21 mmol) der Verbindung aus Beispiel 9f) wurden 368.0 mg der Titelverbindung erhalten.
Fp.: 218-220°C
R_{f} (SiO₂, EE/n-Heptan) = 0.12
MS (DCI): 391

### h) 8-[2-Cyano-6-methoxy-3-(N-ethylaminocarbonyl-glycyl-N-methyl)aminobenzyloxy]-2-methylchinolin

80.0 mg (0.20 mmol) der Verbindung aus Beispiel 9g) wurden mit Ethylisocyanat nach dem in Beispiel 1h) angeführten Verfahren umgesetzt. Es wurden 70.0 mg der Titelverbindung isoliert.
Fp.: 109-111°C
R_{f} (SiO₂, CH₂Cl₂/MeOH 18:2) = 0.15
MS (FAB) : 462 (M+H)⁺

### Beispiel 10 (Vergleichsbeispiel)

### 8-[3-(N-(4-trans-Trifluormethylcinnamoylglycyl)-N-methylamino)-2,6-dimethylbenzyloxy]-2-methylchinolin

### a) 2,6-Dimethyl-3-nitro-benzoesäure

In eine auf 0°C gekühlte Lösung von 25 ml konz. Schwefelsäure und 25 ml 65 %ige HNO₃ wurden portionsweise 13.5 (g (90.0 mmol) 2,6-Dimethylbenzoesäure eingetragen. Nach 1h Rühren bei 0°C wurde die Reaktionsmischung auf Eis gegossen und der ausfallende Niederschlag abgesaugt und getrocknet. Es resultierten 15.5 g der Titelverbindung .
Fp: 109°C
MS (DCI) : 196 (M+H)⁺

### b) 2,6-Dimethyl-3-nitro-benzylalkohol

4,2 g (20.4 mmol) Benzyltriethylammoniumborhydrid in 30 ml CH₂Cl₂ wurden bei 0°C mit 2,6 ml (20.4 mmol) Trimethylchlorsilan versetzt. Nach 15 Min Rühren bei 0°C tropfte man zu dieser Reaktionslösung eine Lösung von 2.0 g (10.2 mmol) der Verbindung aus Beispiel 10a) in 10 ml CH₂Cl₂. Nach 2 h Rühren wurde die Reaktionslösung auf eine 5 %ige NaHCO₃-Lösung gegossen und anschließend 3x mit EE extrahiert. Die vereinten organischen Phasen wurden mit 5 %iger NaHCO₃-Lösung, Wasser und gesättigter NaCl-Lösung gewaschen, über MgSO₄ getrocknet und eingeengt. Hochvakuumtrocknung ergab 700.0 mg der Titelverbindung .
R_{f} (SiO₂, EE) = 0.31
MS (DCI) : 182 (M+H)⁺.

### c) 2,6-Dimethyl-3-nitro-benzyltrifluoromethansulfonat

Unter einer Argon-Atmosphäre wurde eine Lösung von 340.0 mg (1.80 mmol) der Verbindung aus Beispiel 10b) mit 200.0 mg (1.80 mmol) Triethylamin und 230.0 mg (18 mmol) Methansulfonsäurechlorid versetzt. Nach 30 Min Rühren wurde die Reaktionsmischung auf Wasser gegossen und mehrfach mit CH₂Cl₂ extrahiert. Die vereinten CH₂Cl₂-Extrakte wurden über MgSO₄ getrocknet, eingeengt und der Rückstand im Hochvakuum getrocknet. Es wurden 450.0 mg der Titelverbindung isoliert.
MS (DCI) : 260 (M+H)⁺

### d) 8-(2,6-Dimethyl-3-nitro-benzyloxy)-2-methylchinolin

Eine Lösung von 270.0 mg (1.70 mmol) 8-Hydroxy-2-methylchinolin, 450.0 mg (1.70 mmol) der Verbindung aus Beispiel 10c) und 570.0 mg (1.70 mmol) Cs₂CO₃ in 2 ml abs. DMF wurden über Nacht bei RT gerührt. Die Reaktionslösung wurde mit Methyl-tert-butylether versetzt, 2x mit 2N NaOH gewaschen und über MgSO₄ getrocknet. Einengung und Hochvakuum-Trocknung lieferte 510.0 mg der Titelverbindung.
R_{f} (SiO₂, EE/n-Heptan 1:1) = 0.48
MS (DCI) : 323 (M+H)⁺

### e) 8-(3-Amino-2,6-dimethyl-benzyloxy)2-methylchinolin)

Die Titelverbindung wurde aus der Verbindung des Beispiels 10d) nach dem in Beispiel 1d) angeführten Verfahren hergestellt. Aus 500.0 mg (1.5 mmol) der Verbindung aus Beispiel 10d) wurden 450.0 mg der Titelverbindung erhalten.
R_{f} (SiO₂, EE/Heptan 1:1) =0.21
MS (DCI) : 293 (M+H)⁺

### f) 8-[2,6-Dimethyl-3-(N-phthaloylglycyl)amino-benzyloxy]-2-methylchinolin

Die Herstellung der Titelverbindung aus der Verbindung aus Beispiel 10e) erfolgte nach dem in Beispiel 1e) angeführten Verfahren. Aus 440.0 mg (1.4 mmol) der Verbindung aus Beispiel 10e) resultieren 630 mg der Titelverbindung.
R_{f} (SiO₂, EE) = 0.42
MS (DCI) : 480 (M+H)⁺

### g) 8-[2,6-Dimethyl-3-(N-phthaloylglycyl-N-methyl)amino-benzyloxy]-2-methylchinolin

Die Titelverbindung wurde aus der Verbindung des Beispiels 10f) nach dem in Beispiel 1f) angegebenen Verfahren hergestellt. Aus 610.0 mg (1.27 mmol) der Verbindung aus Beispiel 10f) resultierten 480.0 mg der Titelverbindung als schwach-gelbgefärbtes Öl.
R_{f} (SiO₂, EE/n-Heptan 1:1) = 0.19
MS (DCI) = 494 (M+H)⁺

### h) 8-[2,6-Dimethyl-3-(N-glycyl-N-methyl)amino-benzyloxy]-2-methylchinolin

Die Herstellung der Titelverbindung aus der Verbindung aus Beispiel 10g) erfolgte nach dem in Beispiel 1g) angeführten Verfahren. Aus 470.0 mg (0.96 mmol) der Verbindung aus Beispiel 10g) resultierten 180.0 mg der Titelverbindung als schwach gelbgefärbtes Öl.
R_{f} (SiO₂, EE/n-Heptan = 0.14
MS (DCI) : 364 (M+H)⁺

### i) 8-[3-(N-(trans-4-Trifluormethylcinnamoylglycyl)-N-methylamino)-2,6-dimethylbenzyloxy]-2-methylchinolin

Unter einer Argon-Atmosphäre wurde eine Lösung aus 175.0 mg (0.48 mmol) der Verbindung aus Beispiel 10h), 107 mg (0.49 mmol) 4-trans-Trifluormethylzimtsäure, 102.0 mg (0.49 mmol) DCC und 100.0 mg (0.49 mmol) N-Hydroxybenzotriazol in 4 ml abs. DMF über Nacht bei RT gerührt. Die resultierende Reaktionsmischung wurde mit EE verdünnt, mit einer ges. Na₂CO₃- und einer 10%igen NaHSO₄-Lösung gewaschen und über MgSO₄ getrocknet. Das Lösungsmittel wurde im Vakuum entfernt und der Rückstand durch Säulenchromatographie an SiO₂ (EE) gereinigt. Es wurden 50 mg der Titelverbindung als amorpher Feststoff isoliert.
R_{f} (SiO₂, EE) = 0.40
MS (FAB) : 562 (M+H)⁺

### Beispiel 11

### 8-[6-Chloro-2-methoxy-3-(N-(4-trans-trifluoromethylcinnamoyl-glycyl)-N-methylamino)-benzyloxy]-2-methylchinolin

### a) 2-Chlor-6-methoxy- und 6-Chlor-2-methoxy-3-nitro-toluol

Bei 0°C wurde zu einer Suspension von 5.8 g (0,145 mol) Natriumhydrid (60 %ige Suspension in Mineralöl) in 200 ml DMF 5,8 ml (0,145 mol) Methanol hinzugefügt. Nach 30 Min Rühren bei dieser Temperatur wurden portionsweise 30 g (0,145 mol) 2,6-Dichlor-3-nitrobenzol zugegeben, wobei die Temperatur auf 20°C anstieg. Anschließend wurde 1.5 h ohne Kühlung gerührt und nach Zugabe von 300 g Eis 3 x mit Ethylacetat (3 x 800 ml) extrahiert. Die Extrakte wurden über MgSO₄ getrocknet und im Vakuum eingeengt. Säulenchromatographie an SiO₂ mit EE/n-Heptan als Eluens lieferte die beiden Titelverbindungen als Öle.
α) 2-Methoxy-6-chlor-3-nitrobenzol
   Ausbeute: 8.0 g
   R_{f} (SiO₂, EE/n-Heptan 1:2) = 0.4
   MS (DCI) : 202 (M+H)
β) 2-Chlor-6-methoxy-3-nitrobenzol
   Ausbeute: 2.6 g
   R_{f} (SiO₂, EE/n-Heptan 1:2) = 0.25
   MS (DCI) : 202 (M+H)

### b) 2-Methoxy-6-chloro-3-nitrobenzylbromid

Zu einer Lösung von 8.0 g (40.0 mmol) 2-Methoxy-6-chloro-3-nitrotoluol aus Beispiel 11 aα) in 50 ml Chlorbenzol wurde bei 110°C ein Gemisch aus 5.8 g (20.0 mmol) 1,3-Dibrom-5,5-dimethylhydantoin und 0,5 Azo-bis-isobutyronitril portionsweise zugegeben. Nach 1 h wurde nochmals ein Gemisch aus 3.0 g (10 mmol) 1,3-Dibrom-5,5-dimethylhydantoin und 0,2 g Azo-bis-isobutyronitril zugegeben. Nach weiteren 1.5 h ließ man abkühlen und gab 500 ml Ethylacetat zur Reaktionslösung. Das resultierende Gemisch wurde je einmal mit ges. Na₂SO₃-, Na₂CO₃- und NaCl-Lösung gewaschen, getrocknet (MgSO₄) und eingeengt. Es resultierten 10.2 g der Titelverbindung in Form eines amorphen Pulvers.
R_{f} (SiO₂, EE/n-Heptan 1:4) = 0.45
MS (DCI) : m/z = 280 (M+H)

### c) 8-(2-Methoxy-3-nitro-6-chlorobenzyloxy)-2-methylchinolin

Zu einer Lösung von 5.0 g (33,9 mmol) 8-Hydroxy-2-methylchinolin in 65 ml DMF wurden bei RT 10,8 g (33,9 mmmol) Cs₂CO₃ zugegeben. Nach 30 Min Rühren wurden 9.5 g (34.0 mmol) der Verbindung aus Beispiel 11b) zur Reaktionslösung hinzugefügt. Nach 18 h Rühren bei RT wurde mit Wasser versetzt, der angefallene Niederschlag abgesaugt und dieser mit 50 ml EE gewaschen. Trocknung im Hochvakuum ergab 10.4 g der Titelverbindung als amorphes Pulver.
R_{f} (SiO₂, EE/n-Heptan 1:1) = 0.40
MS (FAB) : m/z = 359 (M+H)

### d) 8-(2-Methoxy-3-amino-6-chlorobenzyloxy)-2-methylchinolin

4.9 g (13,7 mmol) der Verbindung aus Beispiel 11c) in 60 ml EE wurden mit 15.0 g (66,6 mmol) SnCl₂ x 2 H₂O versetzt und die erhaltene Suspension 1 h bei 70°C gerührt. Nach dem Abkühlen auf RT wurde im Vakuum eingeengt und der erhaltene Rückstand anschließend mit 100 ml 20 %iger NaOH-Lösung versetzt. Mehrmalige Extraktion mit CH₂Cl₂, Trocknung der vereinten organischen Phasen über CaCl₂ und deren Einengung lieferte 4,2 g der Titelverbindung.
R_{f} (EE/n-Heptan 1:1) = 0.15
MS (DCI) : m/z = 329 (M+H)

### e) 8-(2-Methoxy-3-phthaloylglycylamino-6-chlorobenzyloxy)-2-methylchinolin

3,3 g (10 mmol) der Verbindung aus Beispiel 11d) und 1,2 g (10 mmol) DMAP in 30 ml N-Methylpyrrolidon und 10 ml Pyridin wurden mit 3,4 g (15.0 mmol) Phthaloylglycylchlorid versetzt. Die Mischung wurde 1.5 h auf 50°C erhitzt, dann auf 0°C abgekühlt und anschließend 30 ml H₂O zugesetzt. Der ausgefallene Niederschlag wurde abgesaugt und mit 100 ml EE gewaschen. Es resultiert 4.3 g der Titelverbindung als amorphes Pulver.
R_{f} (SiO₂, EE/n-Heptan 1:1 ) = 0,10
MS (FAB) : m/z = 516 (M+H)

### f) 8-[2-Methoxy-3-(N-methyl-N-phthaloylglycyl)-amino-6-chlorobenzyloxy]-2-methylchinolin

Zu einer Lösung von 3,7 g (7,1 mmol) der Vereinbarung aus Beispiel 11e) in 40 ml DMF wurden 313,0 mg (8 mmol) Natriumhydrid (60 %ige Suspension) bei 0°C hinzugegeben. Nach 30 Min wurden 0,5 ml (8.0 mmol) Methyljodid zugespritzt. Anschließend wurde 1h bei RT gerührt, dann auf 0°C gekühlt und 75 ml H₂O zugegeben. Der ausgefallene Niederschlag wurde abgesaugt und mit 30 ml kalten Methanol gewaschen. Es wurden 3.3 g der Titelverbindung isoliert.
R_{f} (SiO₂, EE/n-Heptan 1:1) = 0.12
MS (FAB) = m/z = 530 (M+H)⁺

### g) 8-[2-Methoxy-3-(N-methyl-N-glycyl)amino-6-chlorobenzyloxy]-2-methylchinolin

Eine Lösung aus 1,4 g (2,8 mmol) der Verbindung aus Beispiel 11f) und 0,54 ml (11,2 mmol) Hydrazinhydrat in 60 ml Ethanol wurde 12 h bei RT gerührt. Es wurde eingeengt, 40 ml CH₂Cl₂ zugegeben, filtriert und der feste Rückstand mit 40 ml CH₂Cl₂ extrahiert. Einengung der CH₂Cl₂-Lösung lieferte 0.9 g der Titelverbindung als blaßgelben Schaum.
R_{f} (SiO₂, EE/CH₃OH 1:1) = 0.20
MS (FAB) : m/z = 400 (M+H)

### h) 8-[6-Chloro-2-methoxy-3-(N-4-trans-trifluoromethylcinnamoylglycyl)-N-methylamino)-benzyloxy]-2-methylchinolin

Die Herstellung der Titelverbindung erfolgte durch Umsatz der Verbindung aus Beispiel 11g) mit der Verbindung aus Beispiel 2a) nach dem in Beispiel 2b) angeführten Verfahren. Aus 250.0 mg der Verbindung aus Beispiel 11g) resultierten 140.0 mg (0.62 mmol) der gewünschten Verbindung als amorpher Feststoff.
R_{f} (SiO₂, EE) = 0.40
MS (DCI) : 598 (M+H)⁺

### Beispiel 12

### 8-[6-Chloro-2-methoxy-3-(N-(3-(2-furyl)acrylglycyl)-N-methyl)amino-benzyloxy]-2-methylchinolin

Die Titelverbindung resultierte aus dem Umsatz der Verbindung aus Beispiel 11g) mit β-Furylacrylsäure nach dem in Beispiel 4) angeführten Verfahren. Aus 200 mg (0.5 mmol) der Verbindung aus Beispiel 11g) wurden 39 mg der Titelverbindung hergestellt.
R_{f} (SiO₂, EE) = 0.20
MS (FAB) : 520 (M+H)⁺

### Beispiel 13

### 8-[6-Chloro-2-hydroxy-3-(N-(trans-4-methylcinnamoylglycyl-N-methyl)aminobenzyloxy]-2-methylchinolin

### a) 8-[6-Chloro-2-methoxy-3-(N-(trans-4-methylcinnamoylglycyl-N-methyl)aminobenzyloxy]-2-methylchinolin

Die Titelverbindung wurde durch Umsatz der Verbindung aus Beispiel 11g) mit Z-4-Methylzimtsäure nach dem in Beispiel 10i) angeführten Verfahren hergestellt. Aus 330 mg (0,83 mmol) der Verbindung aus Beispiel 11g) wurden 200mg der Titelverbindung erhalten.
R_{f} (SiO₂, EE) : 0.22
MS (FAB) : 544 (M+H)⁺

### b) 8-[6-Chloro-2-hydroxy-3-(N-(trans-4-methylcinnamoylglycyl-N-methyl)aminobenzyloxy]-2-methylchinolin

Unter Argon wurde eine Lösung von 200 mg (0.37 mmol) der Verbindung aus Beispiel 13a) und 1,5 ml einer 1M Bortribromidlösung (in CH₂Cl₂) in 10 ml abs. CH₂Cl₂ 72 h an RT gerührt. Anschließend wurd die Reaktionslösung mit 20 ml Ethanol versetzt und dann zur Trockene eingeengt. Der erhaltene Rückstand wurde mit H₂O versetzt und die wässerige Lösung mehrfach mit EE extrahiert. Die vereinten EE-Extrakte wurden mit ges. NaCl-Lsg gewaschen, über MgSO₄ getrocknet und eingeengt. Reinigung des Rückstandes durch Chromatographie an SiO₂ (EE) lieferte 50 mg der Titelverbindung als amorphe Substanz.
R_{f} (SiO₂, EE) = 0.37
MS (FAB) : 530 (M+H)⁺

### Beispiel 14

### 8-[2-Chloro-6-methoxy-3-(N-(4-trans-trifluormethylcinnamoylglycyl-N-methyl)amino)benzyloxy]-2-methylchinolin

### a) 2-Chloro-6-methoxy-3-nitrobenzylbromid

Die Titelverbindung wurde aus der Verbindung aus Beispiel 11aβ) nach dem in Beispiel 11b) angeführten Verfahren hergestellt. Aus 2.2 g (10.9 mmol) der Verbindung aus Beispiel 11aβ) resultierten 2.8 g der Titelverbindung als amorphe Substanz.
R_{f} (SiO₂, EE/Heptan 1:4) = 0.26
MS (DCI) : 280 (M+H)⁺

### b) 8-(2-Chloro-6-methoxy-3-nitro-benzyloxy)-2-methylchinolin

Die Titelverbindung wurde aus der Verbindung aus Beispiel 14a) nach dem in Beispiel 11c) angeführten Verfahren herstellt. Aus 2.7 g (9.6 mmol) der Verbindung aus Beispiel 14a) ergaben sich 2.1 g der Titelverbindung als beige, amorphe Substanz.
R_{f} (SiO₂, EE/Heptan 1:1) = 0.38
MS (DCI) = 359 (M+H)⁺

### c) 8-(2-Chloro-6-methoxy-3-amino-benzyloxy)-2-methylchinolin

Die Titelverbindung wurde aus der Verbindung des Beispiels 14b) nach dem in Beispiel 11d) angeführten Verfahren hergestellt. Aus 1.6 g (4.5 mmol) der Verbindung aus Beispiel 14b) resultierten 0.90 g der Titelverbindung als amorpher, gelber Feststoff.
R_{f} (SiO₂, EE/n-Heptan 1:1) = 0.13
MS (DCI) : 329 (M+H)⁺

### d) 8-[2-Chloro-6-methoxy-3-(N-phthaloylglycyl)amino-benzyloxy]-2-methylchinolin

Die Titelverbindung wurde aus der Verbindung des Beispiels 14c) nach dem in Beispiel 11e) angeführten Verfahren hergestellt. Aus 0,88 g (2.68 mmol) der Verbindung aus Beispiel 14c) wurden 0.81 g der Titelverbindung erhalten.
R_{f} (SiO₂, EE/n-Heptan) = 0.10
MS (DCI) : 516 (M+H)

### e) 8-[2-Chloro-6-methoxy-3-(N-phthaloylglycyl-N-methyl)aminobenzyloxy]-2-methylchinolin

Die Titelverbindung wurde aus der Verbindung des Beispiels 14d) nach dem in Beispiel 11f) angeführten Verfahren hergestellt. Aus 0.8 g (1.6 mmol) der Verbindung aus Beispiel 14d) resultierten 0.46 g der Titelverbindung.
R_{f} (SiO₂, EE/n-Heptan 2:1) = 0.17
MS (DCI) : 530 (M+H)⁺

### f) 8-[2-Chloro-6-methoxy-3-(N-glycyl-N-methyl)aminobenzyloxy]-2-methylchinolin

Die Titelverbindung wurde aus der Verbindung des Beispiels 14e) nach dem in Beispiel 11g) angeführten Verfahren hergestellt. Aus 0.45 g (0,85 mmol) der Verbindung aus Beispiel 14e) wurden 220 mg der Titelverbindung in Form eines gelben Öls isoliert.
R_{f} (SiO₂, EE/MeOH 1:1) = 0.05
MS (DCI) : 400 (M+H)⁺

### g) 8-[2-Chloro-6-methoxy-3-(N-(4-trans-trifluormethylcinnamoylglycyl-N-methyl)amino)-benzyloxy]-2-methylchinolin

Die Titelverbindung wurde aus der Verbindung des Beispiels 14f) nach dem in Beispiel 2b) angeführten Verfahren hergestellt. Aus 200 mg (0.50 mmol) der Verbindung des Beispiels 14f) resultierten 60.0 mg der Titelverbindung als amorpher Feststoff.
R_{f} (SiO₂, EE) = 0.31
MS (FAB) = 598 (M+H)⁺

### Beispiel 15

### 8-[2-Chloro-6-methoxy-3-(N-(3-(2-furyl)acrylglycyl)-N-methyl)amino-benzyloxy]-2-methylchinolin

Die Titelverbindung resultierte aus dem Umsatz der Verbindung aus Beispiel 14f) mit β-Furylacrylsäure nach dem in Beispiel 10i) angeführten Verfahren. Aus 150 mg (0.38 mmol) der Verbindung aus Beispiel 14f) wurden 35 mg der Titelverbindung als amorpher Feststoff erhalten.
R_{f} (SiO₂, EE) = 0.19
MS (ESI) : 520 (M+H)⁺

### Beispiel 16

### 8-[2-Methoxy-6-thiomethyl-3-(N-(4-trans-trifluormethylcinnamoylglycyl-N-methyl)amino-benzyloxy]-2-methylchinolin

### a) 8-(2-Methoxy-3-nitro-6-methylthio-benzyloxy)-2-methylchinolin

Unter einer Argon-Atmosphäre wurde eine Lösung von 1.6 g (4.50 mmol) der Verbindung aus Beispiel 11c) in 40 ml abs. DMF mit 320 mg (4.5 mmol) Natriumthiomethylat versetzt. Die Reaktionslösung wurde für 24 h bei RT gerührt und dann mit 20 ml H₂O versetzt. Der ausgefallene Niederschlag wurde abgesaugt, mit 100 ml H₂O gewaschen und 3 h bei 50°C im Vakuum getrocknet. Es resultiert 1.5 g der Titelverbindung.
R_{f} (SiO₂, EE/Heptan 1:1) = 0.37
MS (DCI) : 371 (M+H)⁺

### b) 8-(3-Amino-2-methoxy-6-methylthio-benzyloxy)-2-methylchinolin

Die Titelverbindung wurde aus der Verbindung aus Beispiel 16a) nach dem in Beispiel 11d) angeführten Verfahren hergestellt. Es resultiert aus 1.45 g (4.10 mmol) der Verbindung aus Beispiel 16a) 1.27 g der Titelverbindung.
R_{f} (SiO₂, EE/Heptan 1:1) = 0.29
MS (FAB) : 341 (M+H)⁺

### c) 8-[2-Methoxy-6-methylthio-3-N-phthaloylglycyl)aminobenzyloxy]-2-methylchinolin

Die Titelverbindung wurde aus der Verbindung aus Beispiel 16b) nach dem in Beispiel 11e) angeführen Verfahren hergestellt. Aus 1.20 g (3.53 mmol) der Verbindung aus Beispiel 16b) resultierten 1.35 g der gewünschten Verbindung.
R_{f} (SiO₂, EE/Heptan 2:1) = 0.65
MS (FAB) : 528 (M+H)⁺

### d) 8-[2-Methoxy-6-methylthio-3-(N-phthaloylglycyl-N-methyl)amino-benzyloxy]-2-methylchinolin

Die Herstellung der Titelverbindung aus der Verbindung aus Beispiel 16c) erfolgte nach dem in Beispiel 11f) angeführten Verfahren. Aus 1.3 g (2.5 mmol) der Verbindung aus Beispiel 16c) resultierten 0.80 g der gewünschten Verbindung als amorpher, schwach-gelbgefärbten Feststoff.
R_{f} (SiO₂, EE/n-Heptan 2:1) = 0.25
MS (FAB) : 542 (M+H)⁺

### e) 8-[2-Methoxy-6-methylthio-3-(N-glycyl-N-methyl)aminobenzyloxy]-2-methylchinolin

Die Herstellung der Titelverbindung aus der Verbindung des Beispiels 16d) erfolgte nach dem in Beispiel 11g) angeführten Verfahren. Aus 760 mg (1.50 mmol) der Verbindung aus Beispiel 16d) wurden 390 mg der Titelverbindung als amorpher Schaum erhalten.
R_{f} (SiO₂, EE/n-Heptan 2:1) = 0.04
MS (FAB) : 412 (M+H)⁺

### f) 8-[2-Methoxy-6-thiomethyl-3-(N-(4-trans-trifluormethylcinnamoylglycyl-N-methyl)amino-benzyloxy]-2-methylchinolin

Die Titelverbindung resultierte aus Umsatz der Verbindung aus Beispiel 16e) mit der Verbindung aus Beispiel 2a) nach dem in Beispiel 2b) angeführten Verfahren. Aus 150 mg der Verbindung des Beispiels 16e) wurden 42 mg der Titelverbindung als amorphe Substanz isoliert.
R_{f} (SiO₂, EE) = 0.37
MS (FAB) = 610 (M+H)⁺

### Beispiel 17:

### 8-[2-Methoxy-6-methylthio-3-N-5-methoxycarbonylpentanoyl-glycyl-N-methyl)aminobenzyloxy]-2-methylchinolin

Die Titelverbindung wurde durch Umsatz der Verbindung aus Beispiel 16e) mit Adipinsäuremonomethylester nach dem in Beispiel 4) angeführten Verfahren hergestellt. Aus 200 mg (0.49 mmol) der Verbindung aus Beispiel 16e) resultieren 69 mg der Titelverbindung.
R_{f} (SiO₂, EE) = 0.14
MS (FAB) : 554 (M+H)⁺

### Beispiel 18:

### 8-[6-Methoxy-2-methylthio-3-(3-(6-acetylamino-pyridin-3-yl)acrylglycyl-N-methyl)amino-benzyloxy]-2-methylchinolin

### a) 8-(6-Methoxy-2-methylthio-3-nitro-benzyloxy)-2-methyl-chinolin

Die Herstellung der Titelverbindung erfolgte durch Umsatz der Verbindung aus Beispiel 14b) mit Natriumthiomethylat analog zu dem in Beispiel 16a) beschriebenen Verfahren. Aus 4.0 g (11, 16 mmol) der Verbindung aus Beispiel 14b) wurden 3.0 g der Titelverbindung als amorphes, beiges Pulver erhalten.
R_{f} (SiO₂, EE/n-Heptan 1:1) = 0.19
MS (ESI) : 371 (M+H)⁺

### b) 8-(3-Amino-6-methoxy-2-methylthio-benzyloxy)-2-methylchinolin

Die Titelverbindung wurde aus der Verbindung des Beispiels 18a) nach dem in Beispiel 11d) beschriebenen Verfahren hergestellt. Aus 2.7 g (7.30 mmol) der Verbindung aus Beispiel 18a) wurden 2.3 der gewünschten Verbindung erhalten.
R_{f} (SiO₂, EE/n-Heptan 2:1) = 0.18
MS (DCI) : 341 (M+)⁺

### c) 8-(6-Methoxy-2-methylthio-3-(N-phthaloylglycyl)aminobenzyloxy)-2-methylchinolin

Die Titelverbindung wurde aus der Verbindung des Beispiels 18b) nach dem in Beispiel 11e) beschriebenen Verfahren hergestellt. Aus 2.20 g (6.47 mmol) der Verbindung aus Beispiel 18b) wurden 3.32 g der Titelverbindung synthetisiert.
R_{f} (SiO₂, EE/n-Heptan 2:1) = 0.27
MS (ESI) : 528 (M+H)⁺

### d) 8-(6-Methoxy-2-methylthio-3-(N-phthaloylglycyl-N-methyl)aminobenzyloxy]-2-methylchinolin

Die Herstellung der Titelverbindung aus der Verbindung des Beispiels 18c) erfolgte nach dem in Beispiel 11f) beschriebenen Verfahren. Aus 3.30 g (6.24 mmol) der Verbindung aus Beispiel 18c) resultierten 1.68 g der Titelverbindung als beiger Feststoff.
R_{f} (SiO₂, EE/Heptan 2:1) = 0.20
MS (ESI) : 542 (M+H)⁺

### e) 8-[6-Methoxy-2-methylthio-3-(N-glycyl-N-methyl)aminobenzyloxy]-2-methylchinolin

Die Titelverbindung wurde aus der Verbindung aus Beispiel 18d) nach dem in Beispiel 11g) angeführten Verfahren hergestellt. Aus 1.65 g (3.0 mmol) der Verbindung aus Beispiel 18d) resultierten 810 mg der Titelverbindung als beiger, fester Schaum.
R_{f} (SiO₂, EE/n-Heptan 3:1) = 0.06
MS (DCI) : 412 (M+H)⁺

### f) 8-[6-Methoxy-2-methylthio-3-(3-(6-acetylamino-pyridin-3-yl)acrylglycyl-N-methyl)amino-benzyloxy-2-methylchinolin

Die Titelverbindung resultierte aus dem Umsatz der Verbindung aus Beispiel 18e) mit dem Säurechlorid-Derivat von (E)-3-(6-Acetylamino-3-pyridyl)-acrylsäure (bekannt aus EP-A-622 361, preparation 50) nach dem in Beispiel 2b) beschriebenen Verfahren. Aus 250 mg (0.61 mmol) der Verbindung des Beispiels 18e) wurden 128 mg der Titelverbindung erhalten.
R_{f} (SiO₂, EE/MeOH 10:1) = 0.31
MS (FAB) : 600 (M+H)⁺

### Beispiel 19

### 8-[6-Methoxy-2-methylthio-3-(N-trans-4-trifluormethylcinnamoylglycyl-N-methyl)amino-benzyloxy]-2-methylchinolin

Die Titelverbindung wurde aus der Verbindung aus Beispiel 18e) und der Verbindung aus Beispiel 2a) nach dem in Beispiel 2b) beschriebenen Verfahren synthetisiert. Aus 250 mg (0.61 mmol) der Verbindung 18e) resultierten 70 mg der Titelverbindung als amorphe Substanz.
R_{f} (SiO₂, EE) = 0.25
MS (FAB) : 610 (M+H)⁺

### Beispiel 20

### 8-[6-Methoxy-2-methylthio-3-(N-5-methoxycarbonylpentanoylglycyl-N-methyl)aminobenzyloxy]-2-methylchinolin

Die Titelverbindung wurde durch Umsatz der Verbindung aus Beispiel 18e) mit Adipinsäuremonomethylester nach dem in Beispiel 10i) angeführten Verfahren hergestellt. Aus 300 mg (0,73 mmol) der Verbindung aus Beispiel 18e) resultierten 122 mg der Titelverbindung als amorphe Substanz.
R_{f} (SiO₂, EE/MeOH 1:10) = 0.28
MS (FAB) : 554 (M+H)⁺

### Beispiel 21 (Vergleichsbeispiel)

### 8-[2,6-Dimethoxy-3-(N-(trans-4-trifluormethylcinnamoylglycyl-N-methyl)aminobenzyloxy]-2-methylchinolin

### a) 2,6-Dimethoxy-3-nitro-toluol

11.62 g (76.45 mmol) 2,6-Dimethoxytoluol wurden portionsweise in 40 ml, auf 0°C gekühlte, konzentrierte Salpetersäure eingetragen. Nach 15 Min Rühren bei 0°C wurde die Reaktionslösung auf 250 ml Eis gegossen und 3x mit EE extrahiert. Die vereinten EE-Extrakte wurden über Na₂SO₄ getrocknet, das Lösungsmittel im Vakuum entfernt und der braune ölige Rückstand mittels Säulenchromatographie an SiO₂ (EE/Heptan 1:8) gereinigt. Es wurden 8.59 g der Titelverbindung in Form eines roten Öls isoliert.
R_{f} (SiO₂, EE/Heptan 1:8) = 0.28
MS (DCI) : 198 (M+H)⁺

### b) 2,6-Dimethoxy-3-nitro-benzylbromid

Die Titelverbindung wurde aus der Verbindung des Beispiels 21a) nach dem in Beispiel 1b) beschriebenen Verfahren hergestellt.Aus 8.56 g (43.60 mmol) der Verbindung aus Beispiel 21 a) wurden 9.19 g der Titelverbindung als gelber Feststoff synthetisiert.
Fp.: 69-73°C
R_{f} (SiO₂, EE/n-Heptan 1:4) = 0.30
MS (DCI) : 276/278 (M+H)⁺

### c) 8-(2,6-Dimethoxy-3-nitro-benzyloxy)-2-methylchinolin

Die Titelverbindung wurde durch Umsetzung der Verbindung aus Beispiel 21b) und 8-Hydroxy-2-methylchinolin nach dem in Beispiel 1c) beschriebenen Verfahren hergestellt. Aus 4.0 (14.49 mmol) der Verbindung aus Beispiel 21b) wurden 4.33 g der Titelverbindung als beiger Feststoff erhalten.
Fp: 193-195°C
R_{f} (SiO₂, EE/n-Heptan 1:2) = 0.11
MS (DCI): 355 (M+H)⁺

### d) 8-(3-Amino-2,6-dimethoxy-benzyloxy)-2-methylchinolin

Die Titelverbindung wurde aus der Verbindung 21c) nach dem in Beispiel 1d) beschriebenen Verfahren hergestellt. Aus 4.30 g (12.20 mmol) der Verbindung aus Beispiel 21c) resultierten 2.28 g der Titelverbindung in Form orangefarbener Kristalle.
Fp.: 155-159°C
R_{f} (SiO₂, EE/n-Heptan 2:1 ) = 0.19
MS (ESI) : 325 (M+H)⁺

### e) 8-[2,6-Dimethoxy-3-(N-phthaloylglycyl)amino-benzyloxy]-2-methylchinolin

Die Titelverbindung wurde aus der Verbindung des Beispiels 21d) nach dem in Beispiel 1e) angeführten Verfahren hergestellt. Aus 2.27 g (7.00 mmol) der Verbindung des Beispiels 21d) resultierten 2.46 g der Titelverbindung als gelber Feststoff.
Fp.: 196-199°C
R_{f} (SiO₂, CH₂Cl₂/MeOH 10:1) = 0.72
MS (ESI) : 512 (M+H)⁺

### f) 8-[2,6-Dimethoxy-3-(N-phthaloylglycyl-N-methyl)aminobenzyloxy]-2-methylchinolin

Die Titelverbindung wurde aus der Verbindung aus Beispiel 21e) nach dem in Beispiel 1f) beschriebenen Verfahren hergestellt. Aus 2.45 g (4.81 mmol) der Verbindung aus Beispiel 21e) resultierten 1.2 g der Titelverbindung in Form eines gelbgefärbten, amorphen Feststoffs.
R_{f} (SiO₂, EE/Heptan 2:1 ) = 0.21
MS (ESI): 526 (M+H)⁺

### g) 8-[2,6-Dimethoxy-3-(N-glycyl-N-methyl)amino-benzyloxy]-2-methylchinolin

Die Titelverbindung wurde aus der Verbindung des Beispiels 21f) nach dem in Beispiel 1g) angeführten Verfahren hergestellt. Aus 1.18 g (2.28 mmol) der Verbindung aus Beispiel 21f) resultierten 549 mg der Titelverbindung als fester gelber Schaum.
R_{f} (SiO₂, CH₂Cl₂/MeOH/NH₄OH 10:1:0,1) = 0.34
MS (FAB) : 396 (M+H)⁺

### h) 8-[2,6-Dimethoxy-3-(N-trans-4-trifluormethylcinnamoylglycyl-N-methyl)aminobenzyloxy]-2-methylchinolin

Die Titelverbindung wurde durch Umsatz der Verbindung aus Beispiel 21g) mit der Verbindung aus Beispiel 2a) hergestellt. Aus 90 mg (0.23 mmmol) der Verbindung aus Beispiel 21g) resultierten 74 mg der Titelverbindung als gelbgefärbter amorpher Feststoff.
R_{f} (SiO₂, CH₂Cl₂/MeOH/NH₄OH 10:1:0,1) = 0.67
MS (FAB) : 594 (M+H)⁺

### Beispiel 22

### 8-[6-Methoxy-2-propyloxy-3-(N-(trans-4-trifluormethylcinnamoylglycyl-N-methyl)aminobenzyloxy]-2-methylchinolin

### a) 6-Methoxy-2-propyloxy-3-nitrotoluol

Unter einer Argon-Atmosphäre wurden portionsweise 2,0 g (10.93 mmol) 3-Methoxy-2-methyl-6-nitrophenol (hergestellt nach R.A. Raphael, P. Ravenscoft, J. Chem. Soc. Perkin Trans. I, (1988), 1823-1828) zu einer auf 0°C gekühlten Suspension von 525 mg (12.02 mmol) NaH in 30 ml abs DMF gegeben. Nach 30 Min Rühren bei 0°C wurden 1,17 ml (12.70 mmol) n-Propylbromid hinzugetropft. Die Reaktionslösung wurde 8 h bei 70°C gerührt. Anschließend wurden unter Eiskühlung 70 ml H₂O hinzugefügt und dann zur Trockene eingeengt. Der Rückstand wurde in EE aufgenommen, die Lösung 3x mit H₂O gewaschen, über Na₂SO₄ getrocknet und eingeengt. Trocknung im Hochvakuum ergab 2.37 g der Titelverbindung in Form eines braunen Öls.
R_{f} (SiO₂, EE/n-Heptan 1:3) = 0.45
MS (DCI) : 226 (M+H)⁺

### b) 6-Methoxy-2-propyloxy-3-nitro-benzylbromid

Die Titelverbindung wurde aus der Verbindung des Beispiels 22a) nach dem in Beispiel 1b) angeführten Verfahren hergestellt. Aus 3.27 g (14.53 mmol) der Verbindung aus Beispiel 22a) resultierten 2.77 g der Titelverbindung als brauner Feststoff.
Fp. 53-55°C
R_{f} (SiO₂, EE/n-Heptan 1:3) =
MS (DCl) : 304/306 (M+H)⁺

### c) 8-(6-Methoxy-2-propyloxy-3-nitro-benzyloxy)-2-methylchinolin

Die Titelverbindung wurde durch Umsatz der Verbindung aus Beispiel 22b) und 8-Hydroxy-2-methylchinolin nach dem in Beispiel 1c) angeführten Verfahren hergestellt. Aus 2.77 g (9.11 mmol) der Verbindung aus Beispiel 22b) resultierten 2.73 g der Titelverbindung in Form eines beigen Feststoffs.
Fp.: 159-161 °C
R_{f} (SiO₂, EE/n-Heptan 1:2) = 0.19
MS (ESI) : 383 (M+H)⁺

### d) 8-(3-Amino-6-methoxy-2-propyloxy-benzyloxy)-2-methylchinolin

Die Titelverbindung wurde aus der Verbindung des Beispiels 22c) nach dem in Beispiel 1d) angeführten Verfahren hergestellt. Aus 2.73 g (7.15 mmol) der Verbindung aus Beispiel 22c) resultierten 1.47 g der Titelverbindung als hellbrauner Feststoff.
Fp.: 163-165°C
R_{f} (SiO₂, EE/n-Heptan 2:1) = 0.30
MS (ESI): 353 (M+H)⁺

### e) 8-[6-Methoxy-2-propyloxy-3-(N-phthaloylglycyl)aminobenzyloxy]-2-methylchinolin

Die Titelverbindung wurde aus der Verbindung des Beispiels 22d) nach dem in Beispiel 1e) angeführten Verfahren hergestellt. Aus 1.42 g (4.03 mmol) der Verbindung aus Beispiel 22d) resultierten 1.76 g der Titelverbindung als beiger Feststoff.
Fp.: 200-203°C
R_{f} (SiO₂, CH₂Cl₂/MeOH 20:1) = 0.30
MS (ESI) : 540 (M+H)⁺

### f) 8-[6-Methoxy-2-propyloxy-3-(N-phthaloylglycyl-N-methyl)aminobenzyloxy]2-methylchinolin

Die Titelverbindung wurde aus der Verbindung des Beispiels 22e) nach dem in Beispiel 1f) angeführten Verfahren hergestellt. Aus 1.76 g (3.26 mmol) der Verbindung aus Beispiel 22e) resultierten 1.09 g der Titelverbindung als fester, hellgelber Schaum.
Fp.: 63°C (Erweichung)
R_{f} (SiO₂, EE/n-Heptan 3:1) = 0.35
MS (ESI) : 554 (M+H)⁺

### g) 8-[6-Methoxy-2-propyloxy-3-(N-glycyl-N-methyl)aminobenzyloxy]-2-methylchinolin

Die Titelverbindung wurde aus der Verbindung des Beispiels 22f) nach dem in Beispiel 1g) angeführten Verfahren hergestellt. Aus 1.08 g (1.97 mmol) der Verbindung aus Beispiel 22f) resultierten 915 mg der Titelverbindung als fester, gelber Schaum.
R_{f} (SiO₂, CH₂Cl₂/MeOH/NH₄OH 10:1:0,1) = 0.31
MS (ESI) : 424 (M+H)⁺

### h) 8-[6-Methoxy-2-propyloxy-3-(trans-4-trifluormethylcinnamoylglycyl-N-methyl)amino-benzyloxy]-2-methylchinolin

Die Titelverbindung wurde durch Umsatz der Verbindung aus Beispiel 22g) und der Verbindung aus Beispiel 2a) nach dem in Beispiel 2b) angeführten Verfahren hergestellt. Aus 150 mg (0.36 mmol) der Verbindung aus Beispiel 22g) resultierten 109 mg der Titelverbindung als hellgelbgefärbter Feststoff.
R_{f} (SiO₂, CH₂Cl₂/MeOH/NH₄OH 10:1:0,1) = 0,69
MS (ESI) : 622 (M+H)⁺

### Beispiel 23 (Vergleichsbeispiel)

### 8-[2,6-Dithiomethyl-3-(trans-4-methoxycinnamoylglycyl-N-methyl)amino-benzyloxy]-2-methylchinolin

### a) 8-(2,6-Dithiomethyl-3-nitro-benzyloxy)-2-methylchinolin

Unter einer Argon-Atmosphäre wurde eine Lösung von 5.0 g (13.8 mmol) 8-(2,6-Dichloro-3-nitro-benzyloxy)-2-methylchinolin (bekannt aus EP-A-622 361) und 1,93 (27.6 mmol) Natriumthiomethylat in 50 ml abs. DMF für 48 h bei RT gerührt. Die Reaktionslösung wurde auf Wasser gegossen, die ausgefallenen Kristalle abgesaugt, mit H₂O gewaschen und getrocknet. Reinigung durch Säulenchromatographie an SiO₂ (CH₂Cl₂/EE/Toluol 20:1:5) lieferte 4.4 g der Titelverbindung als gelbe Kristalle.
R_{f} (SiO₂, EE/n-Heptan 1:1 ) = 0.31
MS (ESI) 387 (M+H)⁺

### b) 8-(2-2,6-Dithiomethy)-3-amino-benzyloxy)-2-methylchinolin

Die Titelverbindung wurde aus der Verbindung des Beispiels 23a) nach dem in Beispiel 1d) angeführten Verfahren hergestellt. Aus 4.0 g (10.4 mmol) der Verbindung aus Beispiel 23a) resultierten 3.48 g der Titelverbindung als gelber Feststoff.
R_{f} (SiO₂, EE/n-Heptan 1:1) = 0.25
MS (DCI) : 357 (M+H)⁺

### c) 8-[2,6-Dithiomethyl-3-(N-phthaloylglycyl)amino-benzyloxy]-2-methylchinolin

Die Titelverbindung wurde aus der Verbindung des Beispiels 23b) nach dem in Beispiel 1e) angeführten Verfahren hergestellt. Aus 3.40 g (9.6 mmol) der Verbindung aus Beispiel 23b) resultierten 2,30 g der Titelverbindung in Form eines beigen Schaumes.
R_{f} (SiO₂, EE/n-Heptan 1:1) = 0.28
MS (FAB) : 544 (M+H)⁺

### d) 8-[2,6-Dithiomethyl-3-(N-phthaloylglycyl-N-methyl)amino-benzyloxy]-2-methylchinolin

Die Titelverbindung wurde aus der Verbindung des Beispiels 23c) nach dem in Beispiel 1f) angeführten Verfahren hergestellt. Aus 2,20 g (4.10 mmol) der Verbindung aus Beispiel 23c) resultierten 1.70 g der Titelverbindung als gelber Feststoff.
R_{f} (SiO₂, EE/Heptan 1: 1) = 0.16
MS (FAB) : 558 (M+H)⁺

### e) 8-[2,6-Dithiomethyl-3-(N-glycyl-N-methyl)amino-benzyloxy]-2-methylchinolin

Die Titelverbindung wurde aus der Verbindung des Beispiels 23d) nach dem in Beispiel 1g) angeführten Verfahren hergestellt. Aus 1.60 g (2.90 mmol) der Verbindung aus Beispiel 23d) resultierten 1,06 g der Titelverbindung als beiger, fester Schaum.
R_{f} (SiO₂, EE/MeOH 1:1) = 0.05
MS (FAB) : 428 (M+H)⁺

### f) 8-[2,6-Dithiomethyl-3-(trans-4-methoxycinnamoylglycyl-N-methyl)aminobenzyloxy]-2-methylchinolin

Die Titelverbindung wurde durch Umsatz der Verbindung aus Beispiel 23e) und 4-Methoxyzimtsäure nach dem in Beispiel 10i) angeführten Verfahren hergestellt. Aus 200 mg (0.47 mmmol) der Verbindung aus Beispiel 23e) resultierten 72 mg der Titelverbindung als amorpher Feststoff.
R_{f} (SiO₂, EE) = 0.31
MS (FAB) : 588 (M+H)⁺

### Beispiel 24 (Vergleichsbeispiel)

### 8-[2,6-Dithiomethyl-3-(3-(6-acetylamino-pyridin-3-yl)acryl-glycyl-N-methyl)aminobenzyloxy]-2-methylchinolin

Die Titelverbindung wurde durch Umsatz der Verbindung aus Beispiel 23e) mit dem Säurechlorid-Derivat von (E)-3-(6-Acetylamino-3-pyridyl)-acrylsäure nach dem in Beispiel 2b) beschriebenen Verfahren hergestellt. Aus 150 mg (0.35 mmol) der Verbindung aus Beispiel 23e) resultierten 68 mg der Titelverbindung erhalten.
R_{f} (SiO₂, EE(MeOh 10:1) = 0.33
MS (ESI) : 616 (M+H)⁺

### Beispiel 25

### 8-[2,6-Dichloro-3-[N-(4-Aminobutylaminocarbonylglycyl-N-methyl)amino-benzyloxy]-1,2-dimethyl-1,2,3,4-tetrahydrochinolin-bistrifluoracetat

### a) 1,2-Dimethyl-8-hydroxy-1,2,3,4-tetrahydrochinolin

7,50 g (47.0 mmol) 8-Hydroxy-2-methylchinolin wurden in Gegenwart von 0.75 g PtO₂ in 60 ml Methanol bei 4 bar H₂ und einer Temperatur von 50°C 24 h im Autoklaven hydriert. Die Reaktionslösung wurde filtriert und das Filtrat zur Trockene eingeengt. Der erhaltene Rückstand an 2-Methyl-8-hydroxy-1,2,3,4-tetrahydrochinolin wurde in 60 ml Ethanol gelöst, 3.85 ml 37 %ige FormaldehydLösung und 1,1 g Pd/C (10%ig) hinzugefügt und die erhaltene Reaktionsmischung bei 4 bar H₂ und einer Temperatur von 50°C erneut 24 h im Autoklaven hydriert. Es wurde filtriert, das Filtrat eingeengt und der Rückstand durch Chromatographie an SiO₂ (EE/n-Heptan 1:4) gereinigt. Es wurden 1.56 g der Titelverbindung erhalten.
R_{f} (SiO₂, EE/n-Heptan 1:4) = 0.27
MS (DCI) : 178 (M+H)

### b) 8-(2,6-Dichloro-3-nitro-benzyloxy)-1,2-dimethyl-1,2,3,4-tetrahydrochinolin

Die Titelverbindung wurde durch Umsatz der Verbindung aus Beispiel 25a) und 2,6-Dichloro-3-nitro-benzylbromid (bekannt aus EP-A-622 361) nach dem in Beispiel 1c) angeführten Verfahren hergestellt. Aus 1.4 g (7.90 mmol) der Verbindung aus Beispiel 25a) resultierte 1.20 g der Titelverbindung als amorpher Feststoff.
R_{f} (SiO₂, EE/n-Heptan 1:4) = 0.31
MS (ESI) : 381 (M+H)⁺

### c) 8-(3-Amino-2,6-dichloro-benzyloxy)-1,2-dimethyl-1,2,3,4-tetrahydrochinon

Die Titelverbindung wurde aus der Verbindung des Beispiels 25b) nach dem in Beispiel 1d) angeführten Verfahren hergestellt. Aus 1.15 g (3,03 mmol) der Verbindung des Beispiels 25b) wurden 0,87 g der Titelverbindung erhalten.
R_{f} (SiO₂, EE/n-Heptan 1:1) = 0.17
MS (ESI) : 351 (M+H)⁺

### d) 8-[2,6-Dichloro-3-(N-phthaloylglycyl)amino-benzyloxy]-1,2-dimethyl-1,2,3,4-tetrahydrochinolin

Die Titelverbindung wurde aus der Verbindung des Beispiels 25c) nach dem in Beispiel 1e) angeführten Verfahren hergestellt. Aus 1.10 g (3.14 mmol) der Verbindung aus Beispiel 25c) resultierten 1.27 g der Titelverbindung.
R_{f} (SiO₂, EE/n-Heptan 1:2) = 0.21
MS (ESI) : 538 (M+H)⁺

### e) 8-[2,6-Dichloro-3-(N-phthaloylglycyl-N-methyl)amino-benzyloxy]-1,2-dimethyl-1,2,3,4-tetrahydrochinolin

Die Titelverbindung wurde aus der Verbindung des Beispiels 25d) nach dem in Beispiel 1f) angeführten Verfahren hergestellt. Aus 1.25 g (2,32 mmol) der Verbindung aus Beispiel 25d) resultierten 1.21 g der Titelverbindung.
R_{f} (SiO₂, EE/n-Heptan 2:1) = 0.16
MS (ESI) : 552 (M+H)⁺

### f) 8-[2,6-Dichloro-3-(N-glycyl-N-methyl)amino-benzyloxy]-1,2-dimethyl-1,2,3,4-tetrahydrochinolin

Die Titelverbindung wurde aus der Verbindung des Beispiels 25e) nach dem in Beispiel 1g) angeführten Verfahren hergestellt. Aus 1.20 g (2.17 mmol) der Verbindung aus Beispiel 25e) resultierten 0.67 g der Titelverbindung als amorpher Feststoff.
R_{f} (SiO₂, EE/MeOH 10:1 ) = 0.10
MS (ESI) : 422 (M+H)⁺

### g) 8-[2,6-Dichloro-3-(N-(4-aminobutylaminocarbonyl)glycyl-N-methyl)aminobenzyloxy]-1,2-dimethyl-1,2,3,4-tetrahydrochinolin-bistrifluoroacetat

Die Titelverbindung wurde aus der Verbindung des Beispiels 25f) nach den in den Beispielen 6a und 6b) angeführten Verfahren hergestellt. Aus 0.35 g (0.83 mmol) der Verbindung aus 25f) resultierten 122 mg der Titelverbindung.
R_{f} (SiO₂, CH₂Cl₂/MeOH/NH₄OH 10:1:0,1) = 0.04
MS (FAB) : 536 (M+H)⁺

### Beispiel 26

### 4-Chloro-2-thiomethyl-N-(2-phenyl-ethyl)N-methyl-3-[(2-methylchinolin-8-yl)oxymethyl]benzolsulfonamid

### a) 4-Chloro-2-thiomethyl-N-(1,1-dimethylethyl)-N-methyl-3-[(2-methylchinolin-8-yl)oxymethyl]benzolsulfonamid

Die Titelverbindung wurde aus 2,4-Dichloro-N-(1,1-dimethylethyl)-N-methyl-3-[(2-methylchinolin-8-yl)-oxymethyl]-benzolsulfonamid (bekannt aus WO 96-40639) nach dem in Beispiel 16a) angeführten Verfahren hergestellt.
MS (FAB) : 429 (M+H)⁺

### b) 4-Chloro-2-thiomethyl-N-methyl-3-[(2-methylchinolin-8-yl)oxymethyl]benzolsulfonamid

3.0 g (7,0 mmol) der Verbindung aus Beispiel 26a) wurden in 70 ml 5 N HCl-Lösung 1 h bei RT gerührt. Die Reaktionsmischung wurde auf Eis gegessen und der ausgefallene Niederschlag abgesaugt. Nach Lösung in 2 N NaOH-Lösung wurde mit CH₂Cl₂ extrahiert. Die organische Phase wurde mit H₂O gewaschen, über MgSO₄ getrocknet und zur Trockene eingeengt. Es resultieren 1.9 g der Titelverbindung.
MS (FAB) : 373 (M+H)⁺

### c) 4-Chloro-2-thiomethyl-N-(2-phenyl-ethyl)-N-methyl-3-[(2-methylchinolin-8-yl)oxymethyl]benzolsulfonamid

500.0 mg (1.34 mmol) der Verbindung aus Beispiel 26b) wurden in 5 ml abs. DMF gelöst und mit 64 mg (1.34 mmol) NaH (50 %ig) versetzt. Nach 10 Min Rühren an RT wurden 0,31 g (1.34 mmol) 2-Jodethylbenzol hinzugefügt und 8 h an RT gerührt.

Es wurde Wasser zugesetzt und mehrfach mit EE extrahiert. Die EE-Extrakte wurden mit H₂O gewaschen, über MgSO₄ getrocknet und eingeengt. Reinigung des Rückstands durch Säulenchromatographie an SiO₂ (EE/Heptan 1:5) lieferte 72 mg der Titelverbindung .
MS (FAB) : 477 (M+H)⁺

## Patentansprüche

1. Verbindung der Formel (I) 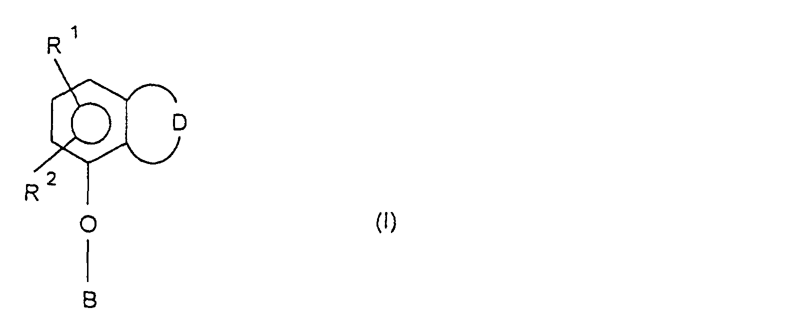 in welcher die Symbole die folgende Bedeutung haben:
D steht für einen Rest der Formel (II) oder (III): 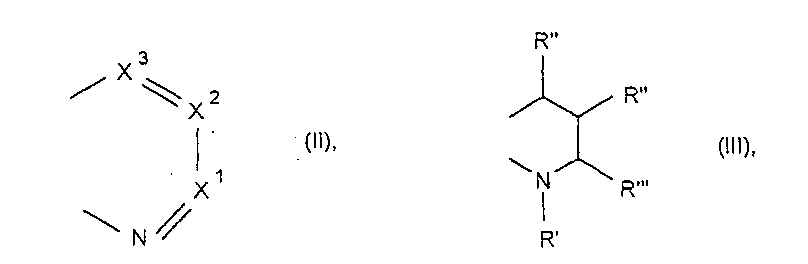
B ein Rest der Formel (IX), 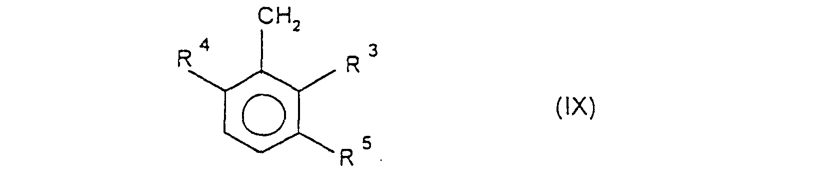
X¹ C-CH₃,
X² C-H,
X³ C-H,
R' Methyl,
R" Wasserstoff,
R''' Methyl,
R¹, R² Wasserstoff,
R³ und R⁴ verschieden sind und bedeuten
1. Chlor,
2. Cyano,
3. Methyl,
4. O-Methyl,
5. S-Methyl,
6. OH,
7. Tetrazolyl, oder
8. CONH₂,
R⁵
1. Nitro,
2. Amino,
3. ein Rest der Formel (X) 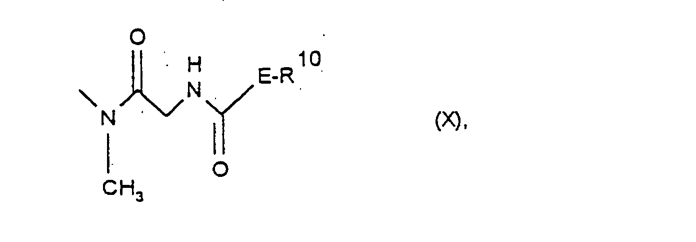
4. ein Rest der Formel (V) 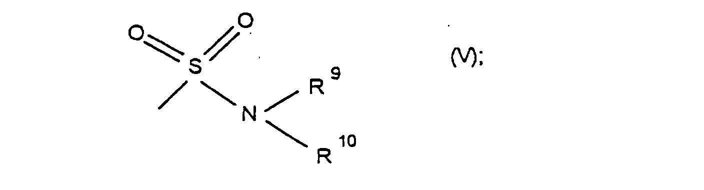
R⁹
1. Wasserstoff,
2. Methyl, Ethyl, n- und i-Propyl und -Butyl,
3. Benzyl
E
1. (C₂-C₅)-Alkandiyl,
2. (C₁-C₇)-Alkandiyl,
3. -(CH₂)ₘ-Tₒ-(CH₂)ₙ-,
wobei die unter 1.-3. aufgeführten Reste gegebenenfalls substituiert sind mit einer Gruppe aus der Reihe OR¹², CO₂R⁹, NR¹³R¹⁴ oder CONR¹³R¹⁴:
T
1. O,
2. NH;
m,n gleich oder verschieden eine Zahl von 0 bis 3;
o eine Zahl von 0 oder 1;
R¹⁰
1. Wasserstoff,
2. (C₁-C₅)-Alkyl,
3. Phenyl,
4. Benzyl,
5. (C₅-C₇)-Heteroaryl, bevorzugt Furyl und Pyridyl
wobei 3., 4. und 5. gegebenenfalls mit einer oder 2 Gruppen aus der Reihe (C₁-C₅)-Alkyl, (C₁-C₅)-Alkoxy, CF₃, OCF₃, NR¹³R¹⁴, NR¹³CO-R¹⁶, CO₂R⁹ substituiert sein können;
R¹¹
1. CF₃,
2. OCF₃;
R¹², R¹³ gleich oder verschieden
1. Wasserstoff,
2. Methyl, Ethyl,
3. Phenyl,
4. Benzyl;
R¹⁴
1. Wasserstoff,
2. C(O)-O-C(CH₃)₃
3. C(O)-O-CH₂-Phenyl
R¹⁶
1. Methyl, Ethyl,
2. Phenyl,
3. (C₅-C₇)-Heteroaryl
wobei diese Reste gegebenenfalls mit einer oder zwei Gruppen aus der Reihe NR¹³R¹⁴ oder CO₂R⁹ substituiert sein können;
sowie deren physiologisch verträgliche Salze.

2. Verbindung der Formel (I) gemäß Anspruch 1 in welcher D für die Formel (II) steht und die übrigen Reste und Variablen wie oben definiert sind.

3. Verfahren zur Herstellung einer Verbindung der Formel (I) gemäß Anspruch 1, das **dadurch gekennzeichnet ist, daß** man
a) eine Verbindung der Formel (XI) 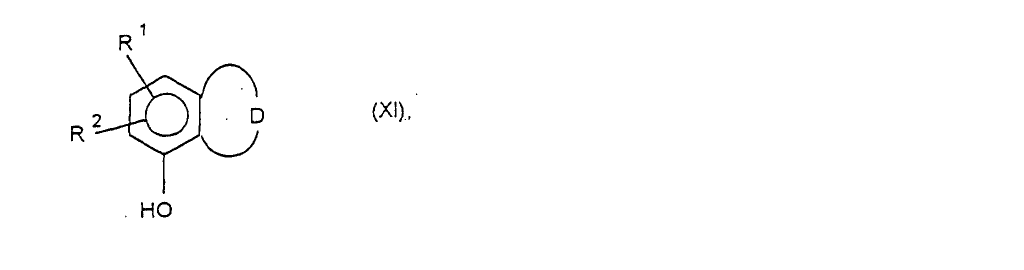 worin R¹, R² und D wie oben definiert sind, in Gegenwart von Metallhydriden oder Alkalimetallcarbonaten in einem inerten Lösungsmittel bei Temperaturen von 0°C bis 60°C mit einer Verbindung der Formel (XII) 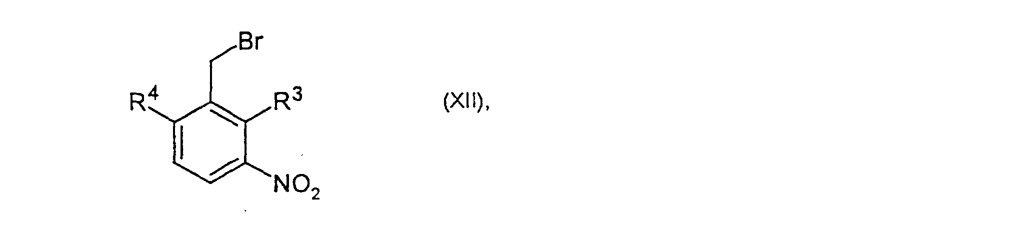 worin R³ und R⁴ wie oben in Formel (I) definiert sind, zu einer Verbindung der Formel (XIII) 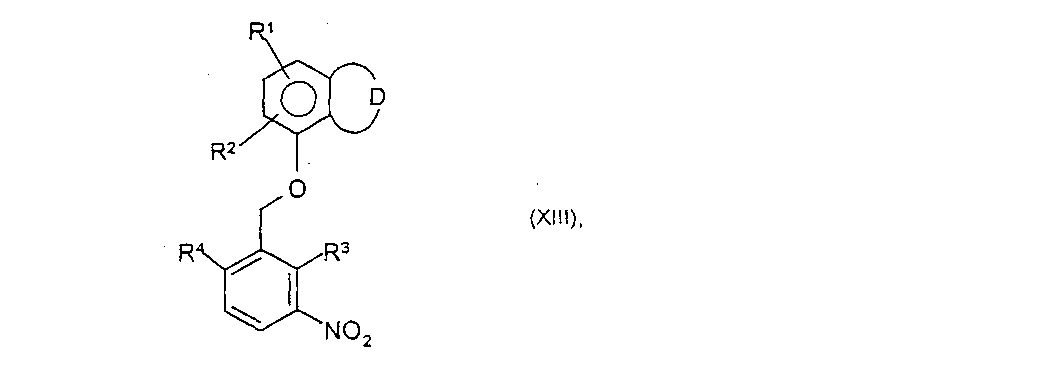 worin R¹, R², R³, R⁴ und D wie oben definiert sind, umsetzt;
b) die Verbindung der Formel (XIII) mit Hilfe von Übergangsmetallhalogeniden zu einer Verbindung der Formel (XIV) reduziert 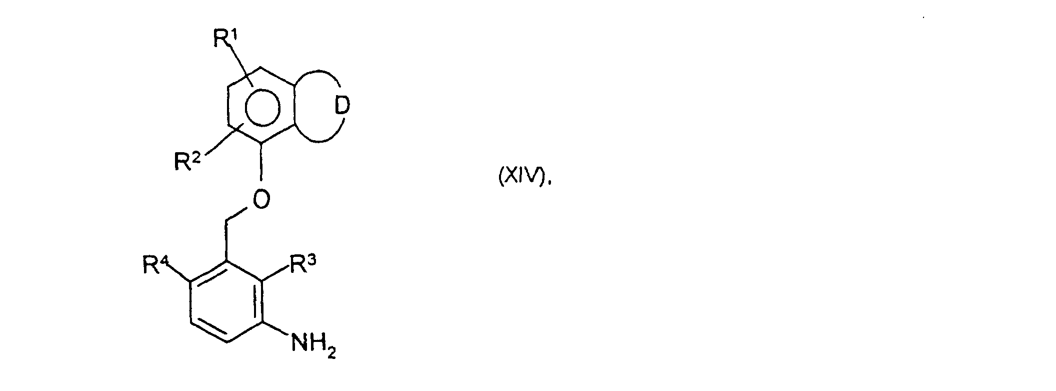 worin R¹, R², R³, R⁴ und D wie oben definiert sind;
c) eine Verbindung der Formel (XIV) mit aktivierten, geeignet geschützten Aminocarbonsäurederivaten von A (= A-Prot) in inerten Lösungsmitteln in Gegenwart einer Base umsetzt und so eine Verbindung der Formel (XV) erhält 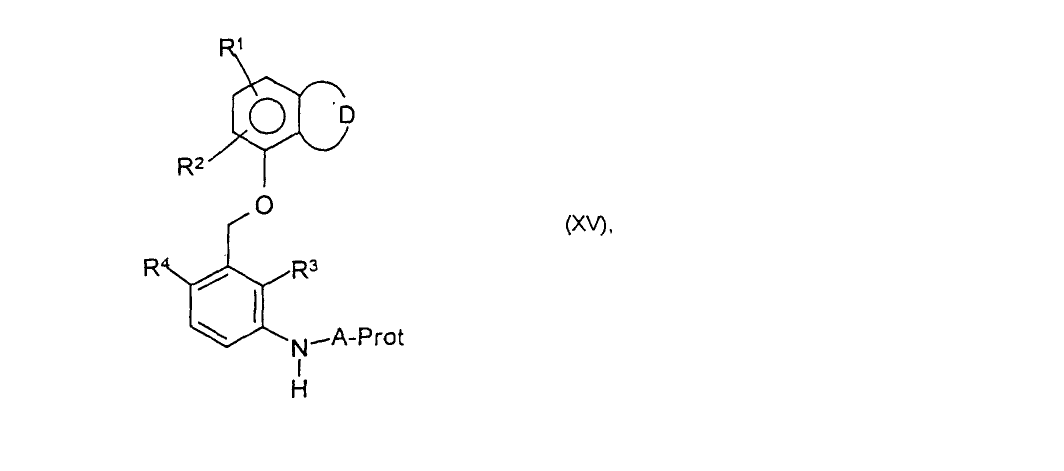 worin A, R¹, R², R³, R⁴ und D wie oben definiert sind, und Prot für eine Aminoschutzgruppe steht;
d) eine Verbindung der Formel (XV) nach erfolgter Einwirkung von Alkalihydriden, Alkalicarbonaten oder Alkoholaten in inerten Lösungsmitteln, gefolgt von einer Behandlung mit R⁹X, wobei R⁹ wie oben definiert ist und X eine Abgangsgruppe bedeutet, umsetzt, wobei man eine Verbindung der Formel (XVI) erhält 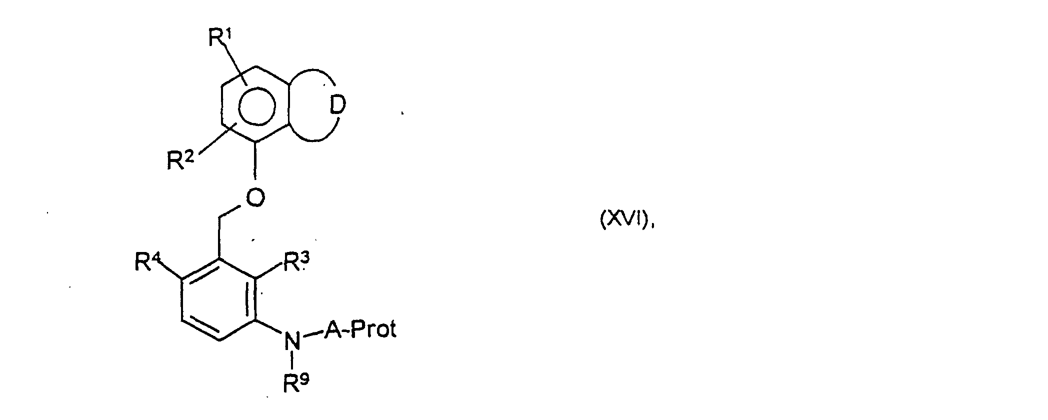 worin R¹, R², R³, R⁴, R⁹, D und A wie oben und Prot wie in Formel (XV) definiert sind;
e) eine Verbindung der Formel (XVI) durch Hydrazinolyse in Ethanol, im Falle der Phthaloylgruppe als Schutzgruppe Prot, bei einer Temperatur von 20°C bis zum Siedepunkt in eine Verbindung der Formel (XVII) 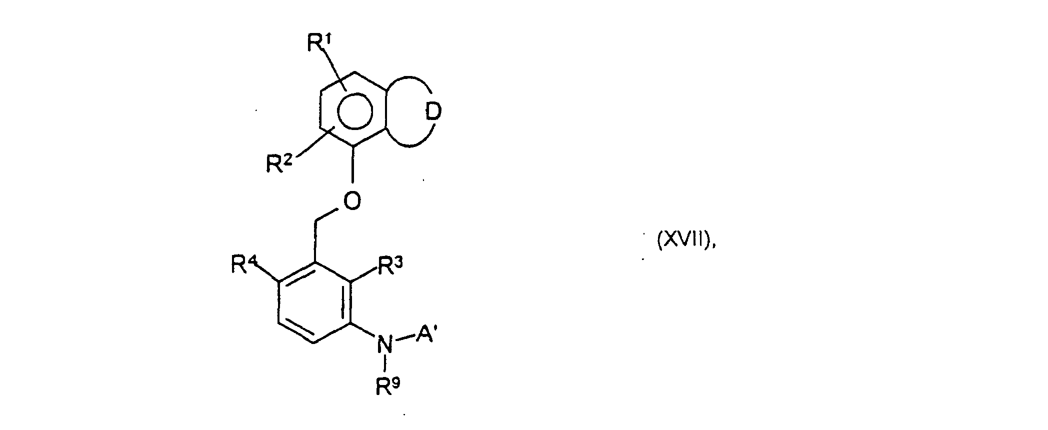 worin R¹, R², R³, R⁴, R⁹ und D wie oben definiert sind und A' für einen Rest einer Aminocarbonsäure steht, überführt;
f1) eine Verbindung der Formel (XVII) mit aktivierten Carbonsäure- und Sulfonsäure-Derivaten R¹⁰-E-Y-OH, worin R¹⁰, E und Y wie oben definiert sind, in konventionellen organischen Lösungsmitteln in Gegenwart einer anorganischen oder organischen Base bei einer Temperatur von 0°C bis Rückfluß zu einer Verbindung der Formel (I), worin R¹, R², R³, R⁴, R⁹, R¹⁰, A, D, E die obengenannte Bedeutung haben, B ein Rest der Formel (VIII) und R⁵ ein Rest der Formel (IV) ist umsetzt, oder
f2) eine Verbindung der Formel (XVII) mit einem Amin oder einem Alkohol R¹⁰-E-NH₂ bzw. R¹⁰-E-OH, in inerten Lösungsmitteln zu einer Verbindung der Formel (I), worin R¹, R², R³, R⁴, R⁹, R¹⁰, A, D, E die obengenannte Bedeutung haben, B ein Rest der Formel (VIII) und R⁵ ein Rest der Formel (IV) ist, umsetzt, wobei man zunächst jedoch die Verbindungen der Formel (XVII) oder das Amin oder den Alkohol mit einer doppelt aktivierten Carbonylverbindung reagieren läßt, oder
f3) eine Verbindung der Formel (XVII) mit einem entsprechenden Isocyanat oder Isothiocyanat in inerten Lösungsmitteln zu einer Verbindung der Formel (I), worin R¹, R², R³, R⁴, R⁹, R¹⁰, A, D, E die obengenannte Bedeutung haben, B ein Rest der Formel (VIII) und R⁵ ein Rest der Formel (IV) ist, umsetzt, und
g) die erhaltenen Verbindungen der Formel (I) gegebenenfalls nach bekannten Methoden in ihre physiologisch verträglichen Salze überführt;
oder
β.
a) eine Verbindung der Formel (XI) 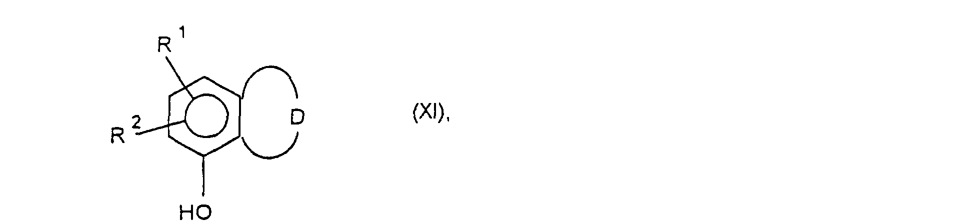 worin R¹, R² und D wie oben definiert sind, in Gegenwart von Metallhydriden oder Alkalimetallcarbonaten in einem inerten Lösungsmittel bei Temperaturen von 0°C bis 60°C mit einer Verbindung der Formel (XVIII) 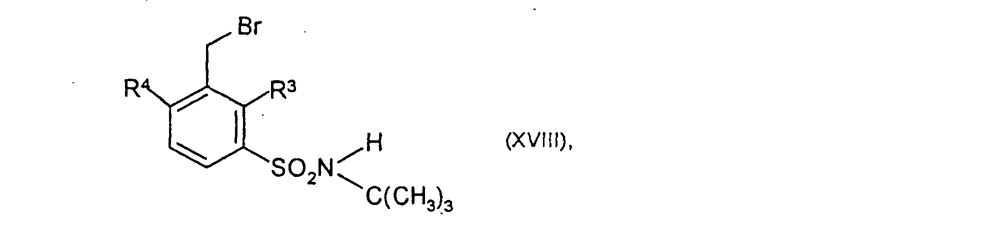 worin R³ und R⁴ wie oben in Formel (I) definiert sind, umsetzt;
b) die aus dieser Verbindung resultierende Verbindung der Formel (XIX) 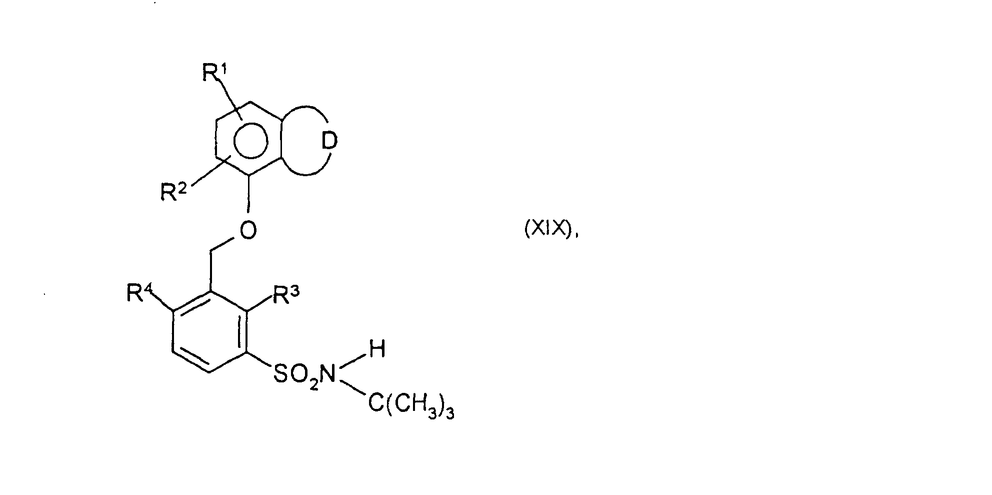 worin R¹, R², R³, R⁴ und D wie oben in Formel (I) definiert sind, in Gegenwart von Metallhydriden in inerten Lösungsmitteln mit Alkyl- oder Arylhalogeniden R⁹-Hal, wobei R⁹ für Alkyl und Aryl wie oben definiert steht, bei einer Temperatur. von 0°C bis 40°C, umsetzt;
c) die resultierende Verbindung der Formel (XX) 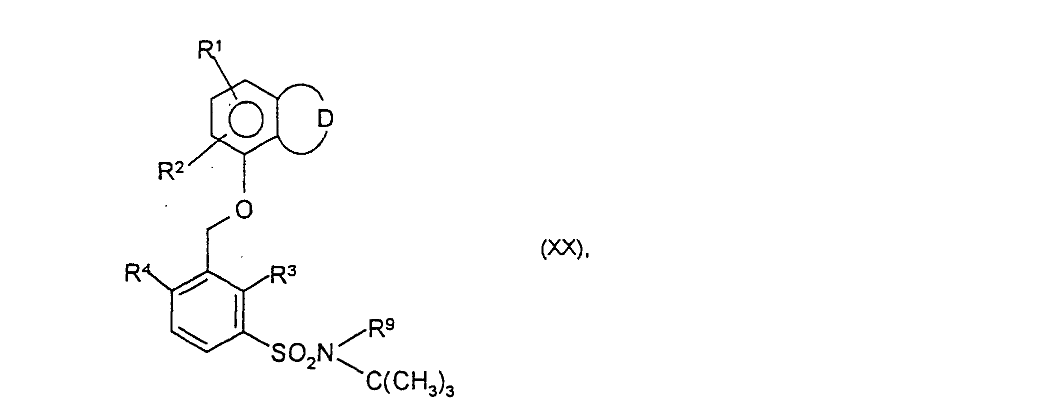 worin R¹, R², R³, R⁴, R⁹ und D wie oben in Formel (I) definiert sind, zunächst mit einem Überschuß an Säure in Gegenwart eines Kationfängers für 4 bis 24 h bei einer Temperatur von 20°C bis 60°C in einem inerten Lösungsmittel behandelt und die erhaltene Verbindung anschließend in Gegenwart einer anorganischen oder organischen Base, mit Halogeniden der Form Hal-R¹⁰, worin R¹⁰ die oben angegebene Bedeutung, ausgenommen Wasserstoff, hat, zu Verbindungen der Formel (I), worin R¹, R², D, R³, R⁴, R⁹ und R¹⁰ die obengenannte Bedeutung haben, B ein Rest der Formel (VIII) und R⁵ ein Rest der Formel (V) ist, umsetzt, und
d) die so erhaltenen Verbindungen der Formel (I) gegebenenfalls in ihr physiologisch verträgliches Salz überführt.

4. Verwendung der Verbindungen der Formel I gemäß den Ansprüchen 1 bis 2 als Heilmittel.

5. Verwendung der Verbindungen der Formel I gemäß den Ansprüchen 1 bis 2 zur Herstellung eines Medikaments zur Behandlung und/oder Prävention der Leberzirrhose.

6. Verwendung der Verbindungen der Formel I gemäß den Ansprüchen 1 bis 2 zur Herstellung eines Medikaments zur Behandlung und/oder Prävention der Alzheimer'schen Krankheit.

7. Heilmittel enthaltend eine wirksame Menge der Verbindungen der Formel I gemäß den Ansprüchen 1 bis 2.

## Claims

1. A compound of the formula (I) 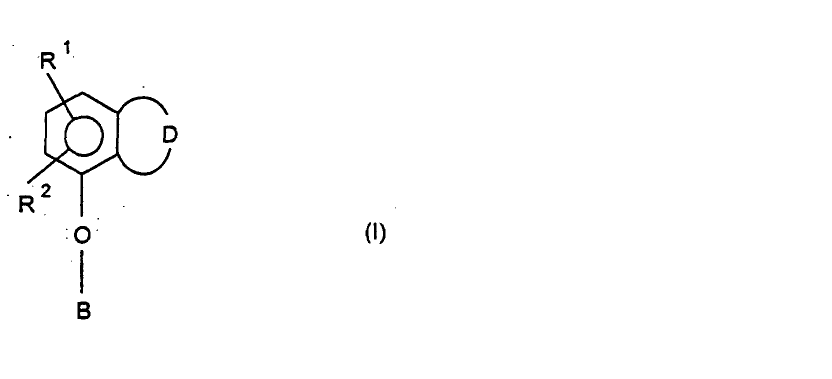 in which the symbols have the following meaning:
D is a radical of the formula (II) or (III): 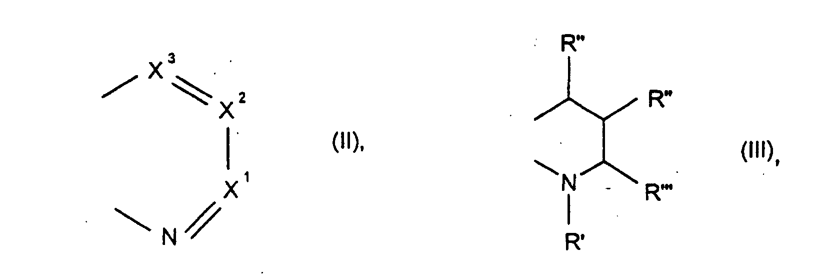
B is a radical of the formula (IX) 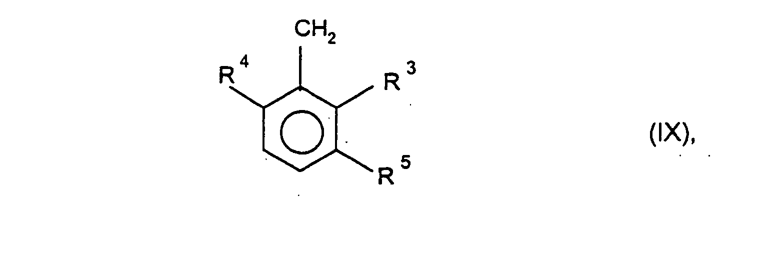
X¹ is C-CH₃,
X² is C-H,
X³ is C-H,
R' is methyl,
R" is hydrogen,
R"' is methyl,
R¹, R² are hydrogen,
R³ and R⁴ are different and are
1. chlorine,
2. cyano,
3. methyl,
4. O-methyl,
5. S-methyl,
6. OH,
7. tetrazolyl, or
8. CONH₂,
R⁵ is
1. nitro,
2. amino,
3. a radical of the formula (X) 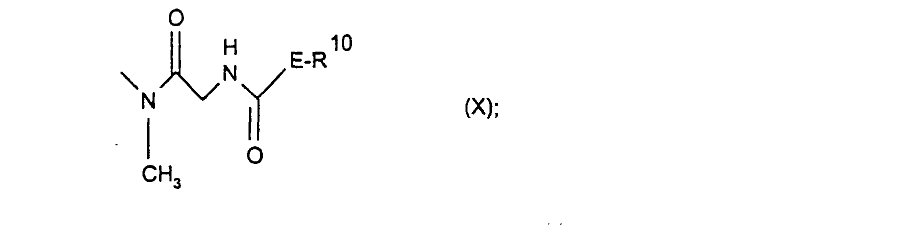
4. a radical of the formula (V) 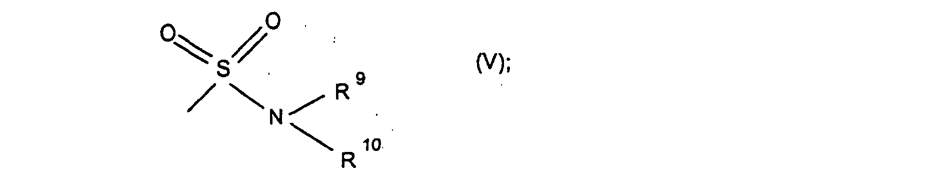
R⁹ is
1. hydrogen,
2. methyl, ethyl, n- or i-propyl or -butyl,
3. benzyl;
E is
1. (C₂-C₅)-alkanediyl,
2. (C₁-C₇)-alkanediyl,
3. -(CH₂)ₘ-Tₒ-(CH₂)ₙ-,
where the radicals mentioned under 1.-3. are optionally substituted by a group of the series OR¹², CO₂R⁹, NR¹³R¹⁴ or CONR¹³R¹⁴;
T is
1. O,
2. NH;
m,n, identically or differently, are a number from 0 to 3;
o is a number 0 or 1;
R¹⁰ is
1. hydrogen,
2. (C₁-C₅)-alkyl,
3. phenyl,
4. benzyl,
5. (C₅-C₇)-heteroaryl, preferably furyl or pyridyl,
where 3., 4. and 5. can optionally be substituted by one or 2 groups of the series (C₁-C₅)-alkyl, (C₁-C₅)-alkoxy, CF₃, OCF₃, NR¹³R¹⁴, NR¹³CO-R¹⁶, CO₂R⁹;
R¹¹ is
1. CF₃,
2. OCF₃;
R¹², R¹³, identically or differently, are
1. hydrogen,
2. methyl, ethyl,
3. phenyl,
4. benzyl;
R¹⁴ is
1. hydrogen,
2. C(O)-O-C(CH₃)₃,
3. C(O)-O-CH₂-phenyl;
R¹⁶ is
1. methyl, ethyl,
2. phenyl,
3. (C₅-C₇)-heteroaryl,
where these radicals can optionally be substituted by one or two groups of the series NR¹³R¹⁴ or CO₂R⁹;
or its physiologically tolerable salts.

2. A compound of the formula (I) as claimed in claim 1, in which D is the formula (II) and the other radicals and variables are,as defined above.

3. A process for the preparation of a compound of the formula (I) as claimed in claim 1, which comprises
a) reacting a compound of the formula (XI) 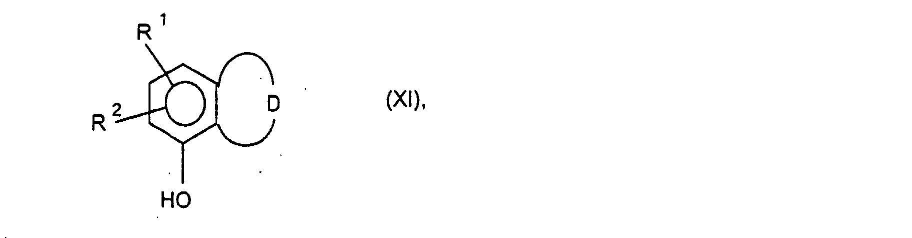 in which R¹, R² and D are as defined above, in the presence of metal hydrides or alkali metal carbonates in an inert solvent at temperatures from 0°C to 60°C with a compound of the formula (XII) 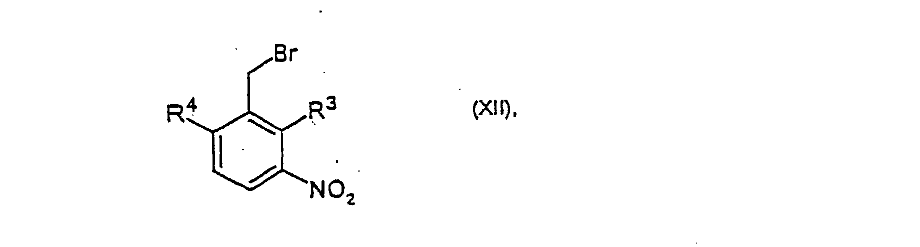 in which R³ and R⁴ are as defined above in formula (I), to give a compound of the formula (XIII) 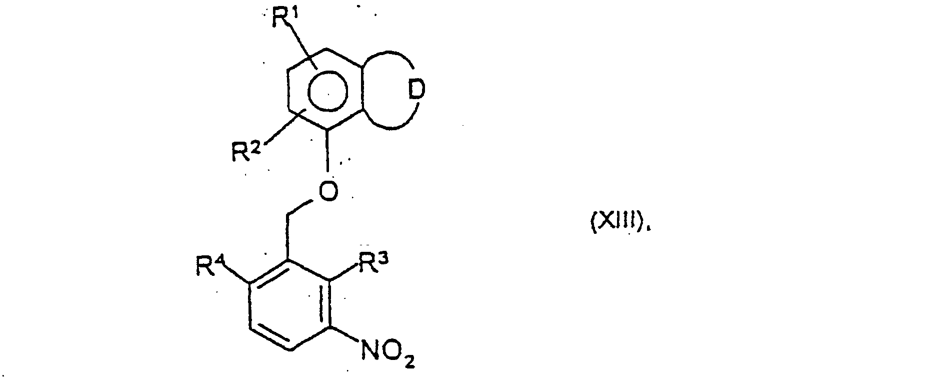 in which R¹, R², R³, R⁴ and D are as defined above;
b) reducing the compound of the formula (XIII) with the aid of transition metal halides to a compound of the formula (XIV) 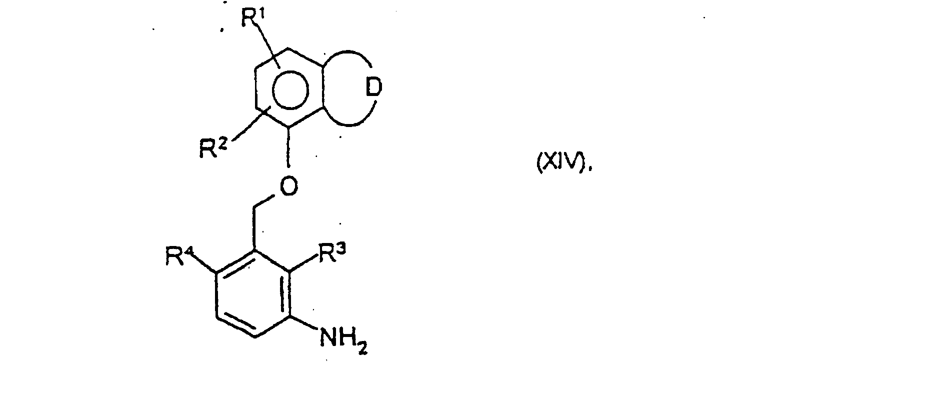 in which R¹, R², R³, R⁴ and D are as defined above;
c) reacting a compound of the formula (XIV) with activated, suitably protected aminocarboxylic acid derivatives of A (= A-Prot) in inert solvents in the presence of a base and thus obtaining a compound of the formula (XV) 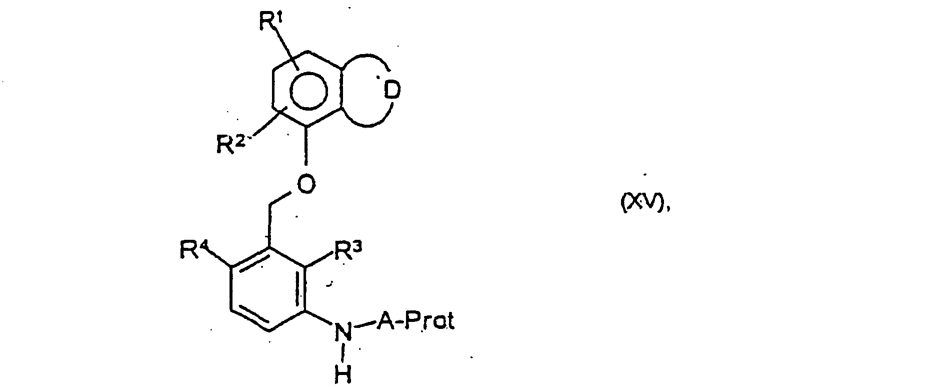 in which A, R¹, R², R³, R⁴ and D are as defined above, and Prot is an amino-protective group;
d) reacting a compound of the formula (XV) after action of alkali metal hydrides, alkali metal carbonates or alcoholates in inert solvents, followed by a treatment with R⁹X, where R⁹ is as defined above and X is a leaving group, a compound of the formula (XVI) being obtained 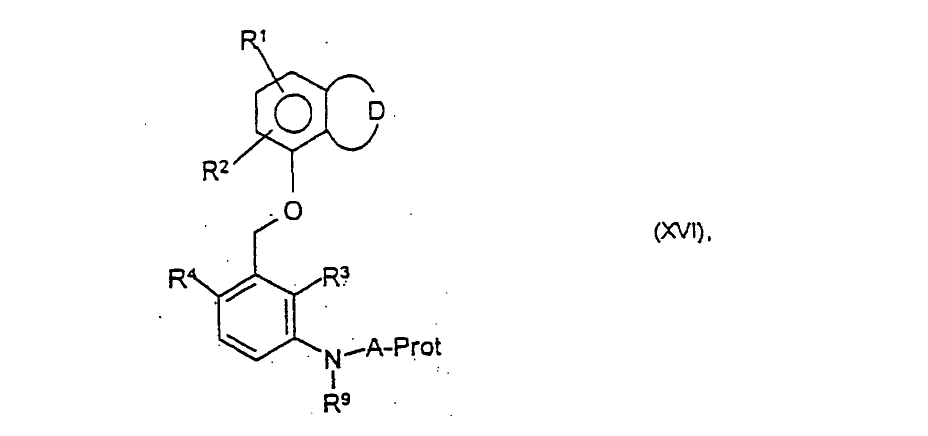 in which R¹, R², R³, R⁴, R⁹, D and A are as defined above and Prot is as defined in: formula (XV);
e) converting a compound of the formula (XVI) by hydrazinolysis in ethanol, in the case of the phthaloyl group as a protective group Prot, at a temperature from 20°C up to the boiling point, into a compound of the formula (XVII) 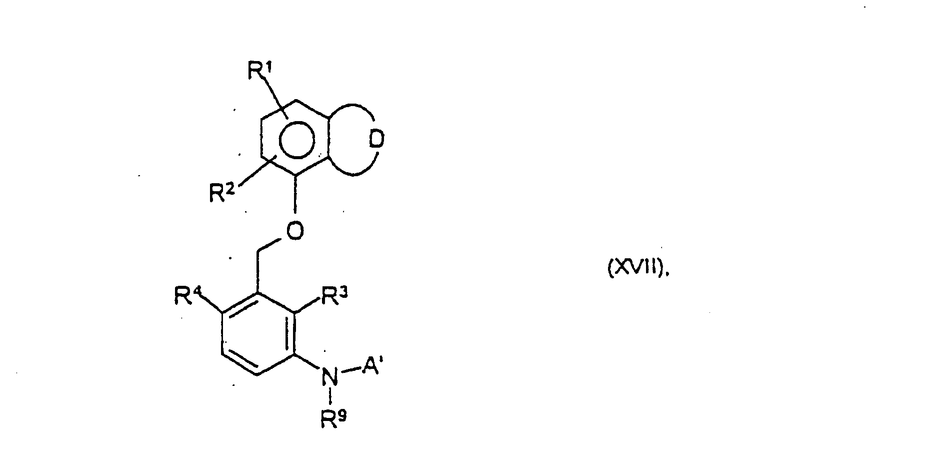 in which R¹, R², R³, R⁴, R⁹ and D are as defined above and A' is a radical of an aminocarboxylic acid;
f1) reacting a compound of the formula (XVII) with activated carboxylic acid and sulfonic acid derivatives R¹⁰-E-Y-OH, in which R¹⁰, E and Y are as defined above, in conventional organic solvents in the presence of an inorganic or organic base at a temperature from 0°C to reflux to give a compound of the formula (I), in which R¹, R², R³, R⁴, R⁹, R¹⁰, A, D, E have the abovementioned meaning, B is a radical of the formula (VIII) and R⁵ is a radical of the formula (IV), or
f2) reacting a compound of the formula (XVII) with an amine or an alcohol R¹⁰-E-NH₂ or R¹⁰-E-OH, in inert solvents to give a compound of the formula (I), in which R¹, R², R³, R⁴, R⁹, R¹⁰, A, D, E have the abovementioned meaning, B is a radical of the formula (VIII) and R⁵ is a radical of the formula (IV), where first, however, the compounds of the formula (XVII) or the amine or the alcohol are allowed to react with a doubly activated carbonyl compound, or
f3) reacting a compound of the formula (XVII) with an appropriate isocyanate or isothiocyanate in inert solvents to give a compound of the formula (I), in which R¹, R², R³, R⁴, R⁹, R¹⁰, A, D, E have the abovementioned meaning, B is a radical of the formula (VIII) and R⁵ is a radical of the formula (IV), and
g) converting the compounds of the formula (I) obtained, if appropriate according to known methods, into their physiologically tolerable salts;
or
β.
a) reacting a compound of the formula (XI) 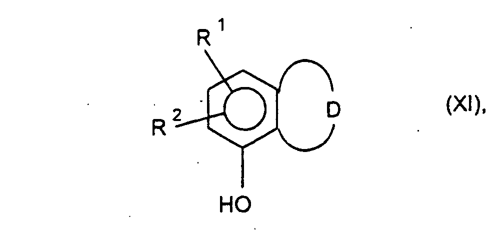 in which R¹, R² and D are as defined above, in the presence of metal hydrides or alkali metal carbonates in an inert solvent at temperatures from 0°C to 60°C with a compound of the formula. (XVIII) 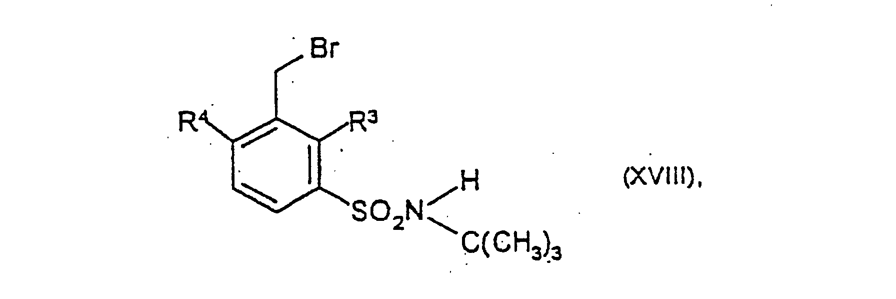 in which R³ and R⁴ are as defined above in formula (I);
b) reacting the compound of the formula (XIX) resulting from this compound 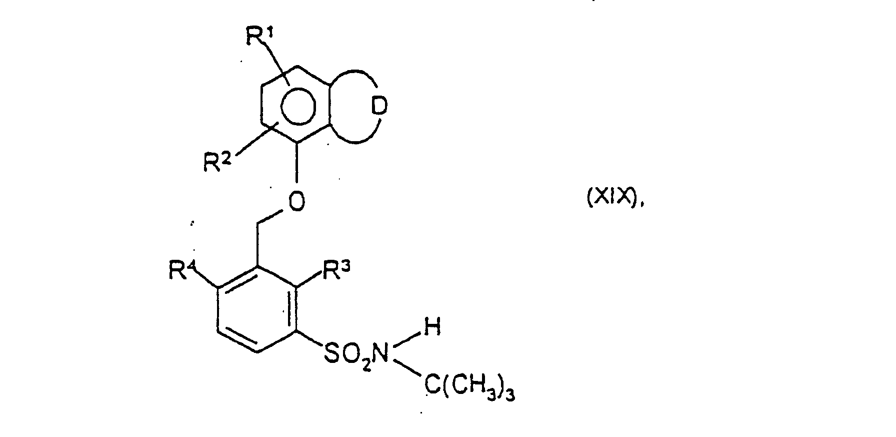 in which R¹, R², R³, R⁴ and D are as defined above in formula (I), in the presence of metal hydrides in inert solvents with alkyl or aryl halides R⁹-Hal, where R⁹ is alkyl and aryl as defined above, at a temperature from 0°C to 40°C;
c) treating the resulting compound of the formula (XX) 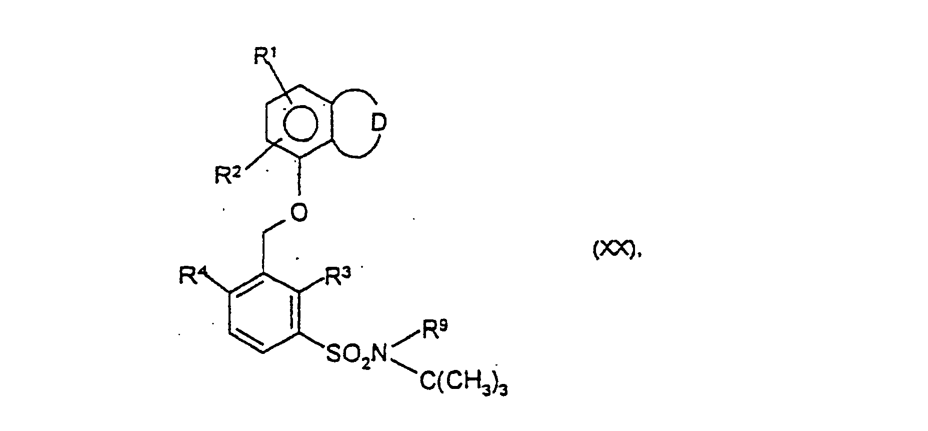 in which R¹, R², R³, R⁴, R⁹ and D are as defined above in formula (I), first with an excess of acid in the presence of a cation scavenger for 4 to 24 h at a temperature from 20°C to 60°C in an inert solvent and then reacting the compound obtained in the presence of an inorganic or organic base, with halides of the formula Hal-R¹⁰, in which R¹⁰ has the meaning indicated above, excluding hydrogen, to give compounds of the formula (I), in which R¹, R², D, R³, R⁴, R⁹ and R¹⁰ have the abovementioned meaning, B is a radical of the formula (VIII) and R⁵ is a radical of the formula (V), and
d) optionally converting the compounds of the formula (I) thus obtained into their physiologically tolerable salts.

4. The use of the compounds of the formula I as claimed in claims 1 and 2 as medicaments.

5. The use of the compounds of the formula I as claimed in claims 1 and 2 for the preparation of a medicament for the treatment and/or prevention of liver cirrhosis.

6. The use of the compounds of the formula I as claimed in claims 1 and 2 for the preparation of a medicament for the treatment and/or prevention of Alzheimer's disease

7. A medicament comprising an efficacious amount of a compound of the formula I as claimed in claims 1 and 2.

## Revendications

1. Composé de formule (I) 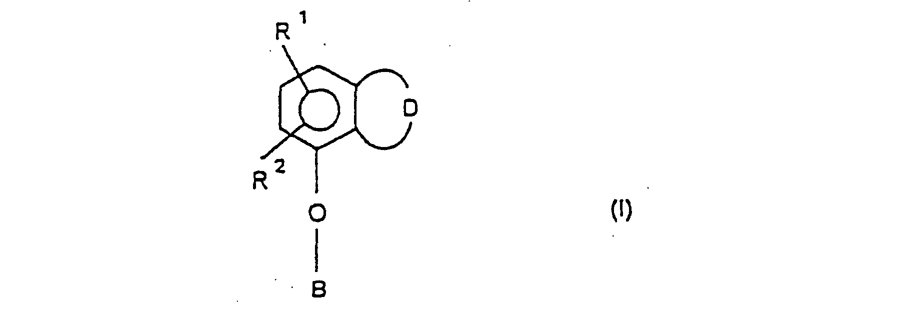 dans laquelle les symboles possèdent les significations suivantes :
D représente un reste de formule (II) ou (III) : 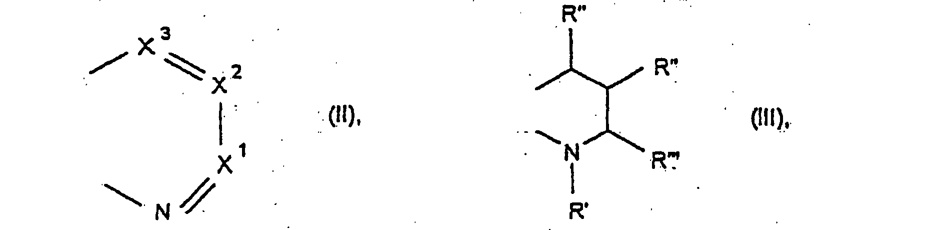
B représente un reste de formule (IX), 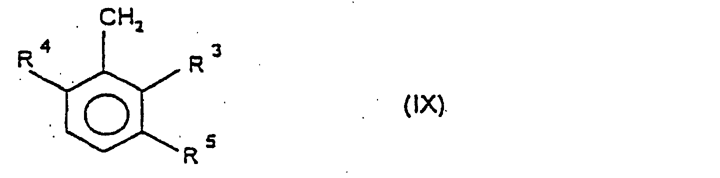
X¹ représente un groupe C-CH₃,
X² représente un groupe C-CH,
X³ représente un groupe C-CH,
R4 représente un groupe méthyle,
R" représente un atome d'hydrogène
R"' représente un groupe méthyle,
R¹, R² représentent un atome d'hydrogène,
R³ et R⁴ sont différents et représentent des groupes
1. chlore,
2. cyano,
3. méthyle,
4. O-méthyle,
5. S-méthyle,
6. OH,
7. tétrazolyle, ou
8. CONH₂,
R⁵ représente un groupe
1. nitro,
2. amino,
3. un reste de formule (X) 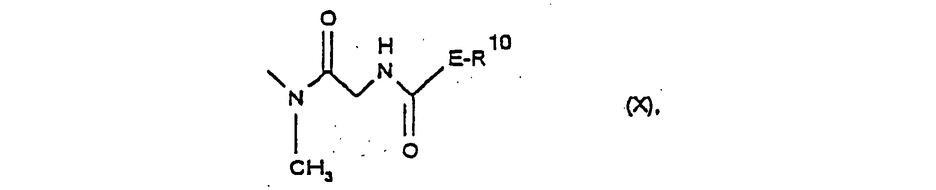
4. un reste de formule (V) 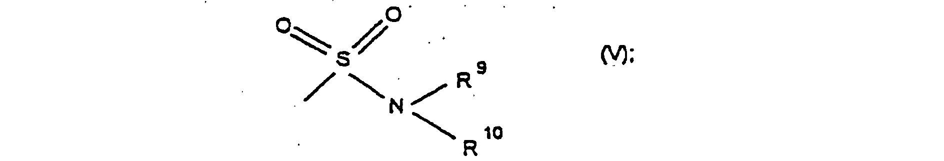
R⁹ représente des groupes
1. hydrogène
2. méthyle, éthyle, n- et i-propyle et -butyle,
3. benzyle
E représente
1. (alcane en C₂-C₅)diyle
2. (alcane en C₁-C₇)diyle
3. -(CH₂)ₘ-Tₒ-(CH₂)ₙ-,
les restes indiqués aux points 1 à 3 étant éventuellement substitués par un groupe par parmi OR¹², CO₂R⁹, NR¹³R¹⁴ ou CONR¹³R¹⁴:
T représente
1. O,
2. NH ;
m,n identiques ou différents, sont un nombre de 0 à 3
o vaut 0 ou 1 ;
R¹⁰ représente
1. un atome d'hydrogène,
2. un groupe alkyle en C₁-C₅,
3. phényle,
4. benzyle,
5. hétéroaryle en C₅-C₇, de préférence furyle et pyridyle,
3., 4. et 5. pouvant éventuellement être substitués par un ou deux groupes pris parmi les substituants alkyle en C₁-C₅, alkoxy en C₁-C₅, CF₃, OCF₃, NR¹³R¹⁴, NR¹³CO-R¹⁶, CO₂R⁹ ;
R¹¹ représente
1. CF₃,
2. OCF₃ ;
R¹², R¹³ identiques ou différents représentent
1. un atome d'hydrogène
2. méthyle, éthyle,
3. phényle,
4. benzyle,
R¹⁴ représente
1. un atome d'hydrogène
2. C(O)-O-C(CH₃)₃
3. C(O)-O-CH₂-phényle,
R¹⁶ représente
1. méthyle, éthyle,
2. phényle,
3. hétéroaryle en C₅-C₇
ces restes pouvant être substitués par un ou deux groupes pris parmi NR¹³R¹⁴ ou CO₂R⁹ ;
ainsi que leurs sels physiologiquement compatibles.

2. Composés de formule (I) selon la revendication 1 dans lesquels D représente la formule (II) et les autres restes et variables sont définis comme ci-dessus.

3. Procédé pour la préparation d'un composé de formule (I) selon la revendication 1, **caractérisés en ce que**
a) on fait réagir un composé de formule (XI) 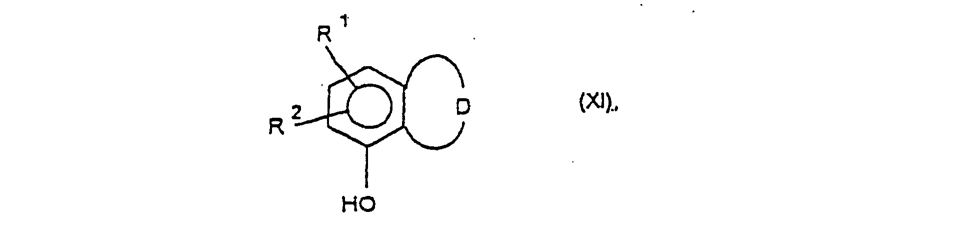 dans laquelle R¹, R² et D sont définis comme ci-dessus, en présence d'hydrures métalliques et de carbonates de métaux alcalins, dans un solvant inerte à des températures de 0 à 60°C, sur un composé de formule (XII) 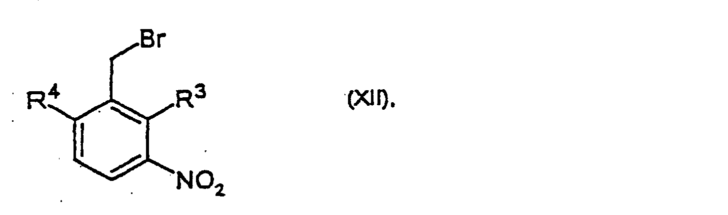 où R³ et R⁴ sont définis comme ci-dessus à la formule (I), pour obtenir un composé de formule (XIII) 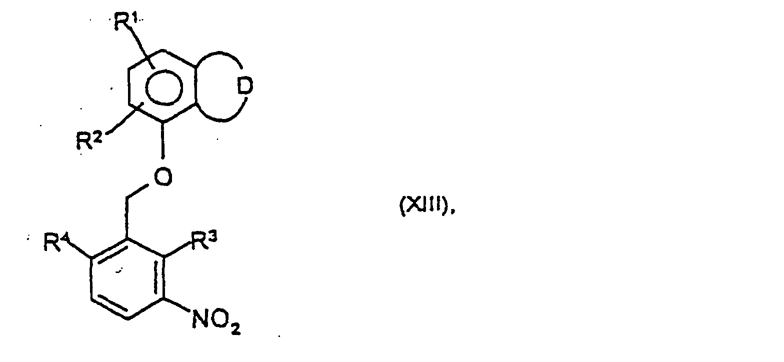 où R¹, R², R³, R⁴ et D sont définis comme ci-dessus ;
b) on réduit le composé de formule (XIII) à l'aide d'halogénures de métaux de transition pour obtenir un composé de formule (XIV) 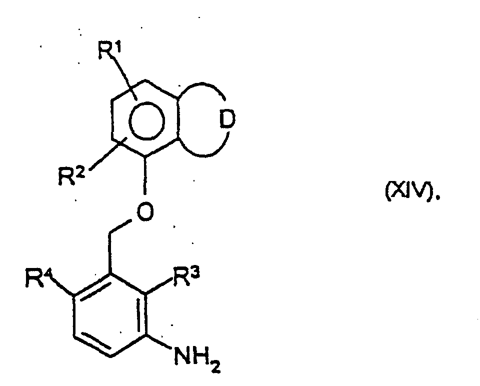 où R¹, R², R³, R⁴ et D sont définis comme ci-dessus ;
c) on-fait réagir un composé de formule (XIV) sur des dérivés d'acides aminocarboxyliques de A (= A-Prot) activés, protégés de façon appropriée, en présence d'une base et on obtient un composé de formule (XV) 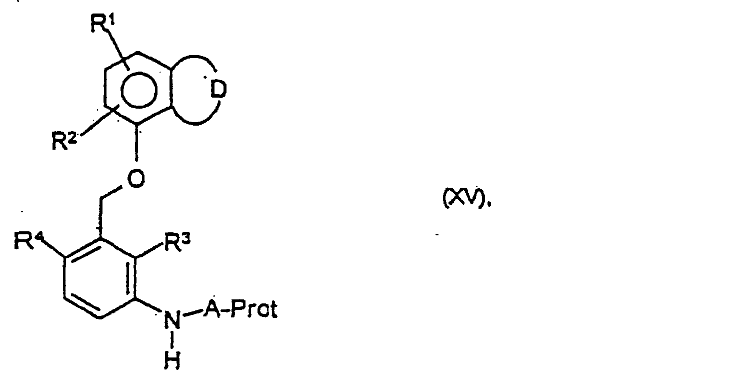 où A, R¹, R², R³, R⁴ et D sont définis comme ci-dessus et Prot représente un groupe protecteur d'amino ;
d) on fait réagir un composé de formule (XV) après avoir fait agir des hydrures alcalins, des carbonates alcalins ou alcoolates dans des solvants inertes, suivi d'un traitement par R⁹X, R⁹ étant défini ci-dessus et X est un groupe partant, en obtenant un composé de formule (XVI) 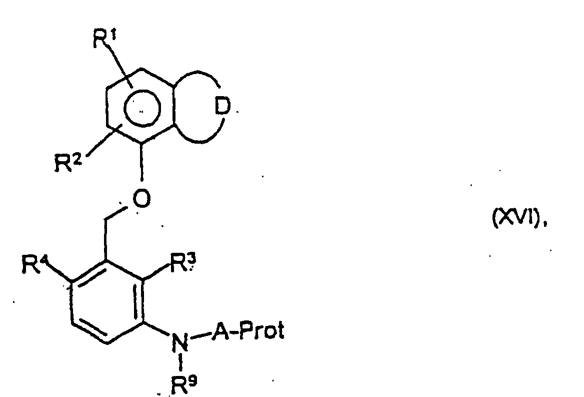 où R¹, R², R³, R⁴, R⁹, D et A sont définis comme ci-dessus et Prot est défini comme à la formule (XV) ;
e) on transforme un composé de formule (XVI) par hydrazinolyse dans l'éthanol, dans le cas du groupe phtaloyle en tant que groupe protecteur Prot, à une température de 20°C au point d'ébullition en un composé de formule (XVII) 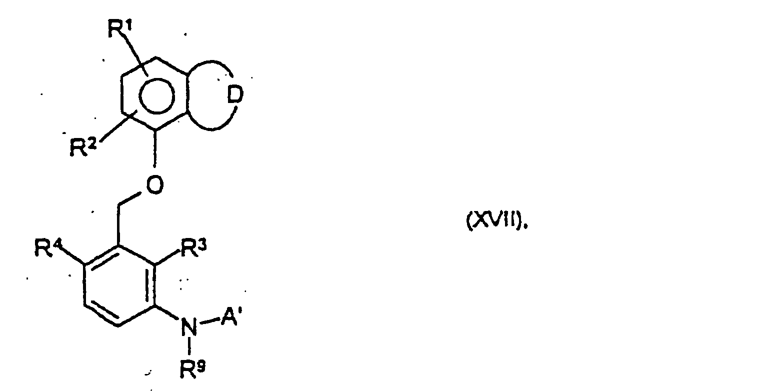 où R¹, R², R³, R⁴, R⁹ et D sont définis comme ci-dessus et A' représente un reste d'un acide aminocarboxylique ;
f1) on fait réagir un composé de formule (XVII) sur des dérivés activés d'acide carboxylique et d'acide sulfonique R¹⁰-E-Y-OH, où R¹⁰, E et Y sont définis comme ci-dessus, dans des solvants organiques classiques, en présence d'une base inorganique ou organique, à une température de 0°C à la température de reflux, pour obtenir un composé de formule (I), où R¹, R², R³, R⁴, R⁹, R¹⁰, A, D, E possèdent les significations données ci-dessus, B représente un reste de formule (VIII) et R⁵ un reste de formule (IV), ou
f2) on fait réagir un composé de formule (XVII) sur une amine ou un alcool R¹⁰-E-NH₂ ou R¹⁰-E-OH, dans un solvant inerte pour obtenir un composé de formule (I), où R¹, R², R³, R⁴, R⁹, R¹⁰, A, D, E possèdent les significations données ci-dessus, B représente un reste de formule (VIII) et R⁵ un reste de formule (IV), toutefois en laissant réagir d'abord les composés de formule (XVII) ou l'amine ou l'alcool sur un composé carbonylé doublement activé,
f3) on fait réagir un composé de formule (XVII) sur un isocyanate ou isothiocyanate dans un solvant inerte pour obtenir un composé de formule (I), où R¹, R², R³, R⁴, R⁹, R¹⁰, A, D, E possèdent les significations données ci-dessus, B représente un reste de formule (VIII) et R⁵ un reste de formule (IV), et
g) on transforme éventuellement les composés obtenus de formule (I) selon des méthodes connues en leurs sels physiologiquement tolérés ;
ou
β.
a) on fait réagir un composé de formule (XI) 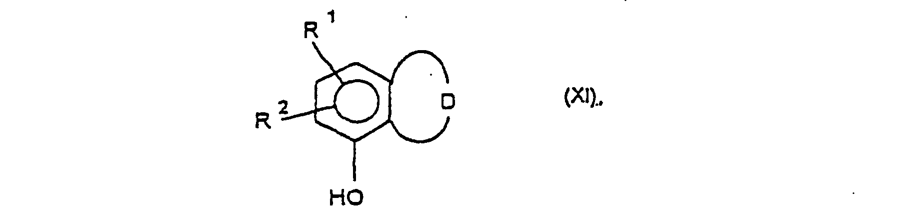 dans laquelle R¹, R² et D sont définis comme ci-dessus, en présence d'hydrures métalliques et de carbonates de métaux alcalins, dans un solvant inerte à des températures de 0 à 60°C, sur un composé de formule (XVIII) 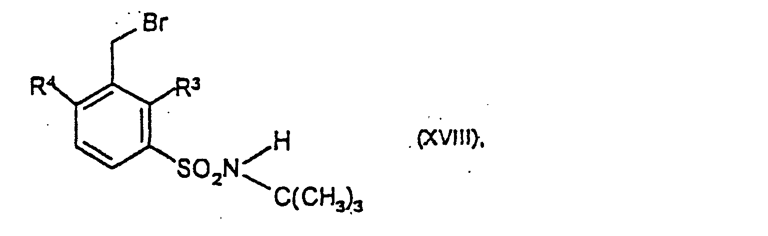 où R³ et R⁴ sont définis comme ci-dessus à la formule (I) ;
b) on -fait réagir le composé de formule (XIX) résultant 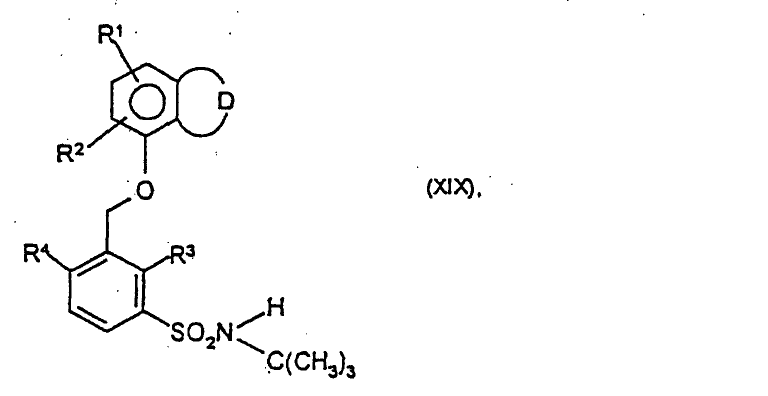 dans laquelle R¹, R², R³, R⁴ et D sont définis comme ci-dessus à la formule (I), en présence d'hydrures métalliques dans un solvant inerte sur des halogénures d'alkyle ou d'aryle R⁹-Hal, R⁹ représente un groupe alkyle ou aryle comme défini ci-dessus, à une température de 0 à 40°C ;
c) on traite le composé résultant de formule (XX) 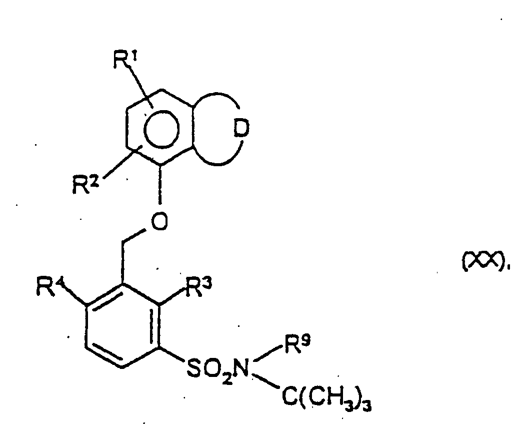 où R¹, R², R³, R⁴, R⁹ et D sont définis comme ci-dessus à la formule (I), d'abord dans un excès en acide en présence d'un capteur de cations pendant 4 à 24 heures, à une température de 20°C à 60°C dans un solvant inerte et ensuite on traite le composé obtenu en présence d'une base organique ou inorganique, sur des halogénures de formule Hal-R¹⁰, où R¹⁰ possède la signification donnée ci-dessus, à l'exception de l'hydrogène, pour obtenir des composés de formule (I), où R¹, R², D, R³, R⁴, R⁹ et R¹⁰ possèdent les significations données ci-dessus, B représente un reste sont de formule (VIII) et R⁵ représente un reste de formule (V), et
d) éventuellement on transforme les composés de formule (I) ainsi obtenus en leur sel physiologiquement toléré.

4. Utilisation des composés de formule (I) selon les revendications 1 à 2 comme médicament.

5. Utilisation des composés de formule (I) selon les revendications 1 à 2 pour la fabrication d'un médicament pour le traitement et/ou la prévention de la cirrhose du foie.

6. Utilisation des composés de formule (I) selon les revendications 1 à 2 pour la fabrication d'un médicament pour le traitement de la maladie d'Alzheimer

7. Médicament renfermant une quantité efficace des composés de formule I selon les revendications 1 à 2.
